# EUROPEAN PATENT SPECIFICATION

(11) **EP 4 370 517 B1**
(45) Date of publication and mention of the grant of the patent: **24.12.2025**
(21) Application number: 22744836.2
(22) Date of filing: 06.07.2022
(51) Int. Cl.: C07D 487/04

(54) **SYNTHESIS OF CHIRAL SUBSTITUTED PYRAZOLOPYRIMIDINE COMPOUNDS**
SYNTHESE CHIRALER SUBSTITUIERTER PYRAZOLOPYRIMIDINVERBINDUNGEN
SYNTHÈSE DE COMPOSÉS DE PYRAZOLOPYRIMIDINE SUBSTITUÉS CHIRAUX

(30) Priority: 12.07.2021 US 202163220820 P
(43) Date of publication of application: 22.05.2024
(73) Proprietor: Galderma Holding SA, 6300 Zug (CH)
(72) Inventor: TOMAS, Loïc, 1814 La Tour de Peilz (CH); HARRIS, Craig Steven, 06410 Biot (FR)
(74) Representative: Impact Intellectual Property LLP
(86) International application number: PCT/IB2022/056257
(87) International publication number: WO 2023/285926

(56) References cited:
- WO-A1-2013/079223
- WO-A1-2019/122059
- OUVRY GILLES ET AL: "Impact of Minor Structural Modifications on Properties of a Series of mTOR Inhibitors", ACS MEDICINAL CHEMISTRY LETTERS, vol. 10, no. 11, 4 October 2019 (2019-10-04), US, pages 1561 - 1567, XP055954445, ISSN: 1948-5875, DOI: 10.1021/acsmedchemlett.9b00401
- OUVRY GILLES ET AL: "Impact of Minor Structural Modifications on Properties of a Series of mTOR Inhibitors - Supporting Information", ACS MEDICINAL CHEMISTRY LETTERS, vol. 10, no. 11, 14 November 2019 (2019-11-14), US, pages 1561 - 1567, XP055954572, ISSN: 1948-5875, DOI: 10.1021/acsmedchemlett.9b00401

## Description

### RELATED APPLICATIONS

This application claims priority under 35 U.S.C. § 119(e) to U.S. Provisional Application No. 63/220820 filed July 12, 2021.

### FIELD

The present technology generally relates to preparation of mTOR inhibitor compounds. Particularly, the present technology relates to a process for synthesis of pyrazolopyrimidine compounds and novel intermediate compounds useful in the process.

### BACKGROUND

The protein kinase mTOR is the catalytic center of two functionally distinct multiprotein complexes, conserved in all eukaryotes and named mTORC1 and mTORC2. mTOR is notably known for regulating cell proliferation, cell growth, cell mobility, cell survival, protein biosynthesis and transcription. mTOR inhibitors such as Rapamycin are useful pharmaceutical compounds for causing disruptions of the mTOR signaling pathway. They are useful for preventing, controlling and treating a variety of diseases and pathological conditions, including dermatological conditions and cancer treatment.

Novel mTOR inhibitor compounds are disclosed in PCT publication WO2019/122059, for example, and have the following general formula (I):

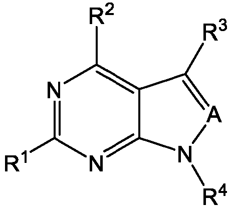

PCT publication WO2019/122065 is also known, which more particularly describes mTOR-inhibiting bicyclic compounds, also used in dermatological complaints and cancer treatment.

The methods described in these patent documents, however, do not describe a feasible production method for producing stereochemically pure compounds because, for example, the methods require the use of expensive reagents and tedious chromatographic purification techniques. Therefore, there is a need in the art for an economical, efficient and simplified method for preparing mTor inhibitors and their synthetic intermediates, which can be scaled for commercial production.

### SUMMARY

In one aspect, a process is provided to produce a pharmaceutical compound represented by the general Formula (I) via a radical cyclization route. The process is completed in fewer steps than the known synthetic methods, and may be conducted to prepare commercially useful quantities. In another aspect, synthetic methods are provided for producing pyrazolopyrimidine derivatives, which are regioselective, efficient, scalable and economical. In another aspect, substantially isomerically pure compounds and intermediates are produced by the above processes. In addition, the present invention includes methods of treating, controlling, delaying or preventing a variety of diseases and pathological conditions, including dermatological complaints and cancer comprising administering the compounds to a subject in need thereof.

According to a first aspect, provided is a process as set out in the appended set of claims of preparing a compound represented by the structural Formula I: 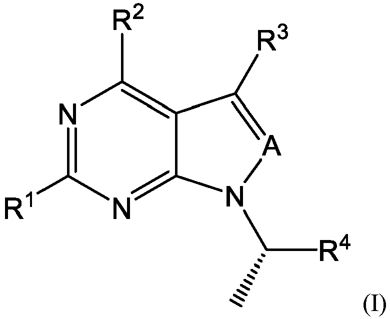 or a hydrate, solvate, or pharmaceutically acceptable salt thereof;
wherein:
A represents -CH or a nitrogen atom;
R¹ represents an -NH₂, -NHMe or -NHEt radical;
R² represents a hydrogen atom, a halogen atom chosen from F, Cl, Br and I,
   an -NH₂, -NHalkyl, -NHacyl, nitrile, methyl (Me), ethyl (Et), trifluoromethyl, -OH or methoxy radical;
wherein when R² is other than an -NH₂ radical, then R¹ represents an -NH₂ radical;
R³ represents a simple or fused bicyclic aromatic or heteroaromatic radical, which is unsubstituted or mono- or polysubstituted with one or more radicals R^{a}; wherein R^{a} is selected from a halogen atom chosen from F, Cl, Br and I, -NH₂, -NHR⁵, nitrile, methyl, ethyl, trifluoromethyl, -OR⁶;
R⁵ represents hydrogen atom, a radical selected from cyclopropyl, acyl, saturated or unsaturated C₁-C₆ alkyl, optionally interrupted with a heteroatom O or S, and unsubstituted or substituted with a C₃-C₅ cycloalkyl or heterocycloalkyl; and
R⁶ represents a hydrogen atom or a methyl radical;
R⁴ represents a hydrogen atom, a linear or branched C₁-C₁₀ alkyl radical, a saturated or unsaturated C₃-C₁₀ ring or bicycle, optionally interrupted with one or more heteroatoms O, S and N, and unsubstituted or substituted with a radical selected from sulfone, fluoro, cyano, ester, -NR⁷, -NR⁷R⁸, C₃-C₆ cycloalkyl or heterocycloalkyl, or an aromatic ring or a heterocycle which is unsubstituted or mono- or polysubstituted with a halogen atom chosen from Cl and F or a radical selected from -OH, -OMe, trifluoromethyl, methyl, ethyl, -NH₂, -NHMe, -NMe₂; and
R⁷ and R⁸ representing, independently of each other, a hydrogen atom, a C₁-C₃ alkyl, cyclopropyl or acyl radical, or together forming a C₃-C₅ ring.

The process of preparing a compound represented by the structural Formula I or a hydrate, solvate, or pharmaceutically acceptable salt thereof comprises:
reacting a compound of Formula II or a salt thereof: 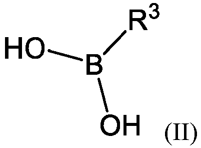
with compound of Formula III or a salt thereof: 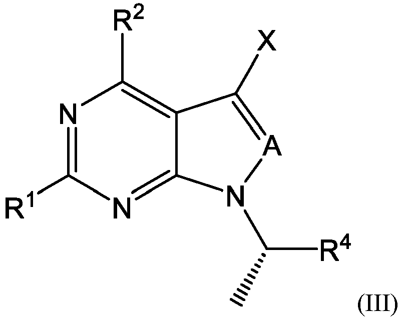
under Suzuki coupling conditions in the presence of a first base to generate a compound of Formula I; and
optionally converting compound of Formula I to a hydrate, solvate, or pharmaceutically acceptable salt thereof.

In one or more embodiments, the compound of Formula II is a hydrochloride salt. In one or more embodiments, the compound of Formula III is an oxalate salt. In one or more embodiments, the X is I. In one or more embodiments, the A is a nitrogen atom. In one or more embodiments, each of R¹ and R² is -NH₂.

In one or more embodiments, R³ is

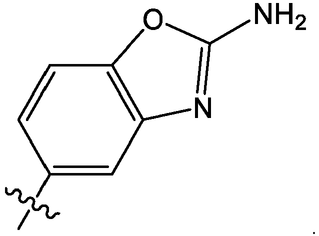

**In** one or more embodiments, R⁴ is an isopropyl radical.

**In** one or more embodiments, the Suzuki coupling conditions comprise heating a reaction mixture comprising the compound of Formula II or a salt thereof, the compound of Formula III or a salt thereof, a Suzuki coupling catalyst, a first base and a solvent. In one or more embodiments, the Suzuki coupling catalyst is selected from the group consisting of PdCl₂(PPh₃)₂, Pd(PPh₃)₄, PdCl₂(dppf)₂, Pd(OAc)₂, Pd₂(dba)₃, or a combination of any two or more thereof. In one or more embodiments, the Suzuki coupling catalyst is Pd(PPh₃)₄. In one or more embodiments, the first base is selected from the group consisting of potassium carbonate, sodium carbonate, potassium bicarbonate, tripotassium phosphate, trisodium phosphate, dipotassium hydrogen phosphate, disodium hydrogen phosphate, or a combination of any two or more thereof. In one or more embodiments, the first base is tripotassium phosphate. In one or more embodiments, the solvent comprises water and isopropyl alcohol. In one or more embodiments, the Suzuki coupling reaction is conducted at a temperature of from about 60 °C to about 120 °C. In one or more embodiments, the reaction is conducted at a temperature of from about 70 °C to about 100 °C.

In one or more embodiments, the compound of Formula II is a hydrochloride salt of compound represented by Formula IIb:

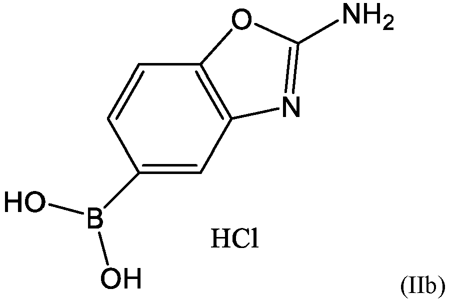

In one or more embodiments, the compound of Formula III is an oxalic acid salt of compound represented by Formula IIIc:

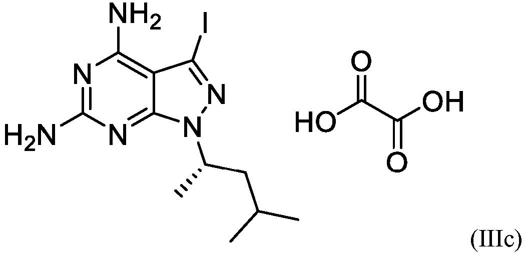

In one or more embodiments, the compound of Formula I is a compound of Formula Ic: 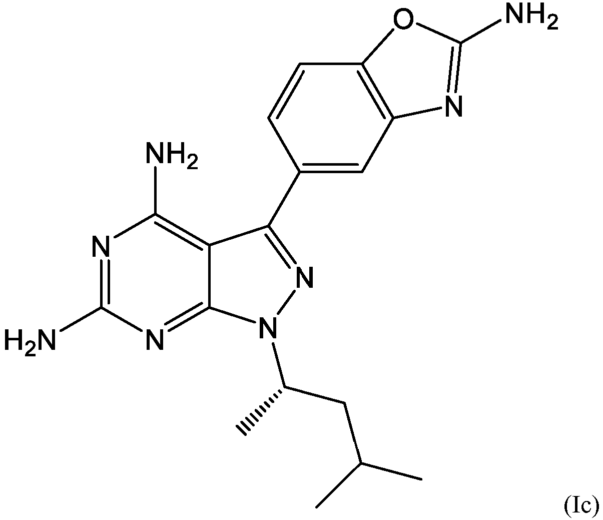 or a hydrate, solvate, or pharmaceutically acceptable salt thereof.

The process further comprises preparing a compound represented by the structural Formula III: 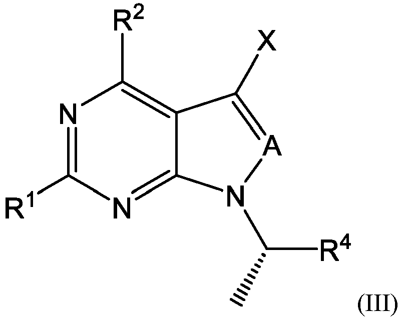
or a salt thereof;
by alkylating a compound of Formula (IV): 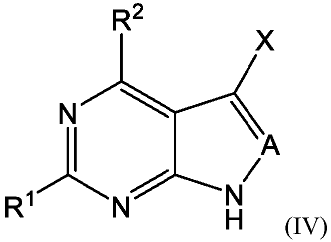
with compound of Formula V: 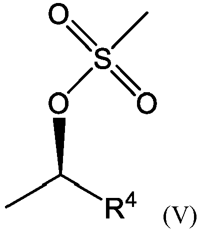
in an anhydrous solvent containing a second base to generate a compound of Formula III.

In one or more embodiments, the second base is selected from the group consisting of cesium carbonate, cesium bicarbonate, cesium hydroxide potassium carbonate, cesium hydride, or a combination of any two or more thereof. In one or more embodiments, the second base is cesium carbonate. In one or more embodiments, the anhydrous solvent is selected from the group consisting of dimethyl sulfoxide, dimethylformamide, dimethylacetamide, N-Methyl-2-pyrrolidone and tetramethylene sulfone or a combination of any two or more thereof. In one or more embodiments, the alkylation reaction is conducted at a temperature of from about 10 °C to about 60 °C. In one or more embodiments, the alkylation reaction is conducted at a temperature of from about 20 °C to about 40 °C. In one or more embodiments, the alkylation reaction is performed for a period of about 5 h to about 30 h. In one or more embodiments, the alkylation reaction is performed for a period of about 8 h to about 20 h.

In one or more embodiments, the process of preparing a compound represented by the structural Formula III, further comprises reacting the compound of Formula III with an acid in a suitable solvent to generate an acid addition salt of compound of Formula III. In one or more embodiments, the acid is oxalic acid and the acid addition salt is the oxalate salt of compound of Formula III. In one or more embodiments, the solvent is selected from the group consisting of ethanol, methanol, isopropanol, butanol, cyclopentyl methylester, methyl acetate, ethyl acetate, isopropyl acetate and mixtures of two or more thereof. In one or more embodiments, the salt formation reaction is conducted at a temperature ranging from 20°C to 70°C. In one or more embodiments, the process further comprises isolating the acid addition salt of compound of Formula III.

In one or more embodiments, the compound of Formula V is prepared by reacting an alcohol compound of Formula VI: 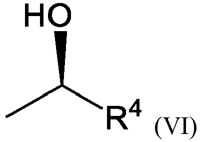 with substituted sulfonyl halide of Formula (IX): 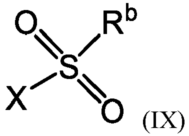
in an organic solvent in the presence of a tertiary amine to generate a compound of Formula V;
wherein:
   X represents F, Cl, Br or I;
   R⁴ represents a hydrogen atom, a linear or branched C₁-C₁₀ alkyl radical, a saturated or unsaturated C₃-C₁₀ ring or bicycle, optionally interrupted with one or more heteroatoms O, S and N, and unsubstituted or substituted with a radical selected from sulfone, fluoro, cyano, ester, -NR⁷, -NR⁷R⁸, C₃-C₆ cycloalkyl or heterocycloalkyl, or an aromatic ring or a heterocycle which is unsubstituted or mono- or polysubstituted with a halogen atom chosen from Cl and F or a radical selected from -OH, -OMe, trifluoromethyl, methyl, ethyl, -NH₂, -NHMe, -NMe₂; and
   R⁷ and R⁸ representing, independently of each other, a hydrogen atom, a C₁-C₃ alkyl, cyclopropyl or acyl radical, or together forming a C₃-C₅ ring; and
   R^{b} represents a hydrogen atom, a linear or branched C₁-C₁₀ alkyl radical or a C₁-C₆ aryl radical.

In one or more embodiments, R⁴ is an isopropyl radical. In one or more embodiments, R^{b} is methyl. In one or more embodiments, the organic solvent is selected from the group consisting of dimethylacetamide, dimethylformamide, toluene, dimethyl sulfoxide, acetonitrile, tetrahydrofuran, dichloromethane, ethyl acetate, methyl ethyl ketone, acetone, and dioxane, or a mixture of any two or more thereof. In one or more embodiments, the tertiary amine is selected from the group consisting of trimethylamine, triethylamine, diisopropylethylamine, dimethylaniline, diethylaniline and dimethylbenzylamine, or a combination of any two or more thereof.

### BRIEF DESCRIPTION OF THE DRAWINGS

**Figure 1** shows supercritical fluid chromatography (SFC) separation and analysis of (S)-3-iodo-1-(4-methylpentan-2-yl)-1H-pyrazolo[3,4-d]pyrimidine-4,6- diamine oxalate using chiral method D.
**Figure 2** shows supercritical fluid chromatography (SFC) separation and analysis of (*S*)-3-(2-aminobenzo[*d*]oxazol-5-yl)-1-(4-methylpentan-2-yl)-1H-pyrazolo[3,4-d]pyrimidine-4,6-diamine (Compound A) using chiral method E.

### DETAILED DESCRIPTION OF THE EMBODIMENTS

Various embodiments are described hereinafter. It should be noted that the specific embodiments are not intended as an exhaustive description or as a limitation to the broader aspects discussed herein. One aspect described in conjunction with a particular embodiment is not necessarily limited to that embodiment and can be practiced with any other embodiment(s).

The use of the terms "a" and "an" and "the" and similar referents in the context of describing the elements (especially in the context of the following claims) are to be construed to cover both the singular and the plural, unless otherwise indicated herein or clearly contradicted by context. Recitation of ranges of values herein are merely intended to serve as a shorthand method of referring individually to each separate value falling within the range, unless otherwise indicated herein, and each separate value is incorporated into the specification as if it were individually recited herein. All methods described herein can be performed in any suitable order unless otherwise indicated herein or otherwise clearly contradicted by context. The use of any and all examples, or exemplary language (*e.g.*, "such as") provided herein, is intended merely to better illuminate the embodiments and does not pose a limitation on the scope of the claims unless otherwise stated. No language in the specification should be construed as indicating any non-claimed element as essential.

The expression "comprising" means "including, but not limited to." Thus, other non-mentioned substances, additives, carriers, or steps may be present. Unless otherwise specified, "a" or "an" means one or more.

Unless otherwise indicated, all numbers expressing quantities of ingredients, reaction conditions, and so forth used in the specification and claims are to be understood as being modified in all instances by the term "about." Accordingly, unless indicated to the contrary, the numerical parameters set forth in the following specification and attached claims are approximations. Each numerical parameter should at least be construed in light of the number of reported significant digits and by applying ordinary rounding techniques. The term "about" when used before a numerical designation, e.g., temperature, time, amount, and concentration, including range, indicates approximations which may vary by ( + ) or ( - ) 10 %, 5% or 1 %.

As used herein, Cₘ-Cₙ, such as *e.g.,* C₁-C₃, C₃-C₅, C₁-C₁₂, C₁-C₈, or C₁-C₆ when used before a group refers to that group containing m to n carbon atoms.

The term "alkyl" refers to monovalent saturated aliphatic hydrocarbyl groups having from 1 to 12 carbon atoms *(i.e.,* C₁-C₁₂ alkyl) or 1 to 8 carbon atoms *(i.e.,* C₁-C₈ alkyl), or 1 to 4 carbon atoms. This term includes, by way of example, linear and branched hydrocarbyl groups such as methyl (CH₃-), ethyl (CH₃CH₂-), n-propyl (CH₃CH₂CH₂-), isopropyl ((CH₃)₂CH-), n-butyl (CH₃CH₂CH₂CH₂-), isobutyl ((CH₃)₂CHCH₂-), sec-butyl ((CH₃)(CH₃CH₂)CH-), t-butyl ((CH₃)₃C-), n-pentyl (CH₃CH₂CH₂CH₂CH₂-), and neopentyl ((CH₃)₃CCH₂-). An alkyl group is optionally substituted with halogen *(e.g.,* -F, -Cl, -Br or -I), an alkoxy, a cycloalkyl *(e.g.,* cyclopentyl or cyclohexyl), an aryl (e.g., phenyl), or heteroaryl group.

The term "acyl" means a radical obtained by removing the hydroxyl group from a carboxylic acid; the acyl group corresponding to a carboxylic acid of formula -RCOOH will have the formula -RCO, in which the carbon atom and the oxygen atom are linked via a double bond (carbonyl group).

The term "alkoxy" refers to an alkyl radical attached through an oxygen linking atom, represented by -O-alkyl. For example, "(C1-C3) alkoxy" includes methoxy, ethoxy and propoxy.

The term "aryl" refers to a monovalent, aromatic mono- or bicyclic ring having 6-10 ring carbon atoms. Examples of aryl include phenyl and naphthyl. The condensed ring may or may not be aromatic provided that the point of attachment is at an aromatic carbon atom.

A phenyl group or a phenoxy group can be optionally substituted with one or more *(e.g.,* two, three, four or five) substituents independently selected from the group consisting of - NO₂, -CN, halogen *(e.g.,* -F, -Cl, -Br or -I), (C1-C3)alkyl, halo(C1-C3)alkyl, (C1-C3)alkoxy and halo(C1-C3)alkoxy.

The term "cycloalkyl" means a saturated aliphatic cyclic hydrocarbon radical optionally containing one or more double bonds. It can be monocyclic, bicyclic, polycyclic (e.g., tricyclic), fused, bridged, or spiro. For example, monocyclic (C₃-C₆)cycloalkyl means a radical having from 3-6 carbon atoms arranged in a monocyclic ring. A (C₃-C₆)cycloalkyl includes, but is not limited to, cyclopropyl, cyclobutyl, cyclopentyl and cyclohexyl. The cycloalkyl is optionally substituted with one or more *(e.g.,* one, two, three, four or five) substituents independently selected from halogen *(e.g.,* -F, -Cl, -Br or -I), an alkyl, an alkoxy, an aryl *(e.g.,* phenyl), or a heteroaryl group.

The terms "halo" or "halogen" or even "halide" can refer to fluoro, chloro, bromo, and iodo.

The term "haloalkyl" means an alkyl group substituted with one or more (e.g., two, three, four, five or six) halogen (-F, -Cl, -Br or -I).

The term "heteroaryl" refers to aromatic ring groups having five to fourteen ring atoms selected from carbon and at least one (typically 1 to 4, more typically 1 or 2) heteroatoms (e.g., oxygen, nitrogen or sulfur). "Heteroaryl" includes monocyclic rings and polycyclic rings in which a monocyclic heteroaromatic ring is fused to one or more other aromatic or heteroaromatic rings.

Examples of monocyclic 5-6 membered heteroaryl groups include furanyl *(e.g., 2-*furanyl, 3-furanyl), imidazolyl *(e.g.,* N-imidazolyl, 2-imidazolyl, 4-imidazolyl, 5-imidazolyl), isoxazolyl *(e.g.,* 3-isoxazolyl, 4-isoxazolyl, 5-isoxazolyl), oxadiazolyl *(e.g.,* 2-oxadiazolyl, 5-oxadiazolyl), oxazolyl *(e.g.,* 2-oxazolyl, 4-oxazolyl, 5-oxazolyl), pyrazolyl *(e.g.,* 3-pyrazolyl, 4-pyrazolyl), pyrrolyl *(e.g.,* 1-pyrrolyl, 2-pyrrolyl, 3-pyrrolyl), pyridyl *(e.g.,* 2-pyridyl, 3-pyridyl, 4-pyridyl), pyrimidinyl *(e.g.,* 2-pyrimidinyl, 4-pyrimidinyl, 5-pyrimidinyl), pyridazinyl *(e.g., 3-*pyridazinyl), thiazolyl *(e.g.,* 2-thiazolyl, 4-thiazolyl, 5-thiazolyl), triazolyl *(e.g.,* 2-triazolyl, 5-triazolyl), tetrazolyl *(e.g.,* tetrazolyl), thienyl *(e.g.,* 2-thienyl, 3-thienyl), and pyridinyl . Examples of polycyclic aromatic heteroaryl groups include carbazolyl, benzimidazolyl, benzothienyl, benzofuranyl, indolyl, quinolinyl, benzotriazolyl, benzothiazolyl, benzoxazolyl, benzimidazolyl, isoquinolinyl, indolyl, isoindolyl, acridinyl, or benzisoxazolyl.

The terms "heterocycle" or "heterocyclic" or "heterocycloalkyl" or "heterocyclyl" refers to a saturated or partially saturated, but not aromatic, group having from 1 to 10 ring carbon atoms and from 1 to 4 ring heteroatoms selected from the group consisting of nitrogen, sulfur, or oxygen. Heterocycle encompasses single ring or multiple condensed rings, including fused bridged and spiro ring systems. In fused ring systems, one or more the rings can be cycloalkyl, aryl or heteroaryl provided that the point of attachment is through the non-aromatic ring. In one embodiment, the nitrogen and/or sulfur atom(s) of the heterocyclic group are optionally oxidized to provide for the N-oxide, sulfinyl, sulfonyl moieties. Examples include a piperidino, morpholino, pyrrolidino or piperazino radical.

Combinations of substituents and variables are only those that result in the formation of stable compounds. The term "stable," as used herein, refers to compounds which possess stability sufficient to allow manufacture and which maintains the integrity of the compound for a sufficient period of time to be useful for the purposes detailed herein.

As used herein, the term "prodrug" means a derivative of a compound that can hydrolyze, oxidize, or otherwise react under biological conditions *(in vitro* or *in vivo)* to provide an active compound. Examples of prodrugs include, but are not limited to, derivatives of a compound that include biohydrolyzable groups such as biohydrolyzable amides, biohydrolyzable esters, biohydrolyzable carbamates, biohydrolyzable carbonates, biohydrolyzable ureides, and biohydrolyzable phosphate analogues *(e.g.,* monophosphate, diphosphate or triphosphate).

As used herein, "hydrate" is a form of a compound wherein water molecules are combined in a certain ratio as an integral part of the structure complex of the compound.

As used herein, "solvate" is a form of a compound where solvent molecules are combined in a certain ratio as an integral part of the structure complex of the compound.

"Pharmaceutically acceptable" means in the present description being useful in preparing a pharmaceutical composition that is generally safe, non-toxic and neither biologically nor otherwise undesirable and includes being useful for veterinary use as well as human pharmaceutical use.

"Pharmaceutically acceptable salts" or "salts thereof" mean salts which are pharmaceutically acceptable, as defined above, and which possess the desired pharmacological activity. Such salts include acid addition salts formed with organic and inorganic acids, such as hydrogen chloride, hydrogen bromide, hydrogen iodide, sulfuric acid, phosphoric acid, acetic acid, glycolic acid, maleic acid, malonic acid, oxalic acid, methanesulfonic acid, trifluoroacetic acid, fumaric acid, succinic acid, tartaric acid, citric acid, benzoic acid, ascorbic acid and the like. Base addition salts may be formed with organic and inorganic bases, such as sodium, ammonia, potassium, calcium, ethanolamine, diethanolamine, N-methylglucamine, choline and the like. Included are pharmaceutically acceptable salts or compounds of any of the Formulae herein.

Depending on its structure, the phrase "pharmaceutically acceptable salt," as used herein, refers to a pharmaceutically acceptable organic or inorganic acid or base salt of a compound. Representative pharmaceutically acceptable salts include, *e.g.,* alkali metal salts, alkali earth salts, ammonium salts, water-soluble and water-insoluble salts, such as the acetate, amsonate (4,4-diaminostilbene-2, 2 -disulfonate), benzenesulfonate, benzonate, bicarbonate, bisulfate, bitartrate, borate, bromide, butyrate, calcium, calcium edetate, camsylate, carbonate, chloride, citrate, clavulariate, dihydrochloride, edetate, edisylate, estolate, esylate, fumarate, gluceptate, gluconate, glutamate, glycollylarsanilate, hexafluorophosphate, hexylresorcinate, hydrabamine, hydrobromide, hydrochloride, hydroxynaphthoate, iodide, isothionate, lactate, lactobionate, laurate, malate, maleate, mandelate, mesylate, methylbromide, methylnitrate, methylsulfate, mucate, napsylate, nitrate, N-methylglucamine ammonium salt, 3-hydroxy-2-naphthoate, oleate, oxalate, palmitate, pamoate (1,1-methene-bis-2-hydroxy-3-naphthoate, einbonate), pantothenate, phosphate/diphosphate, picrate, polygalacturonate, propionate, p-toluenesulfonate, salicylate, stearate, subacetate, succinate, sulfate, sulfosalicylate, suramate, tannate, tartrate, teoclate, tosylate, triethiodide, and valerate salts.

The term "chiral compound" refers to compounds with either a chiral carbon atom, or a configurationally stable chiral heteroatom. Compounds where chirality is caused by restricted rotation or is due to the overall three dimensional shape, *e.g.* a helical shape, and suitable substituted adamantanes are also termed "chiral compounds".

As used herein, substantially pure compound or isomer, *e.g.,* regioisomer, refers to one isomer being 90% of the resulting isomeric mixture, or preferably 95% of the resulting isomeric mixture, or more preferably 98% of the resulting isomeric mixture, or even more preferably 99% of the resulting isomeric mixture, and most preferably above 99% of the resulting isomeric mixture.

The present technology relates to process for preparing mTOR-inhibiting compounds or a pharmaceutically acceptable salt thereof.

The term "mTOR inhibitor" refers to compounds which down-regulate, i.e. reduce, block or even suppress, the activation of the mTOR signaling pathway, by competing, advantageously selectively, with the substrates at the level of mTORC1 and/or mTORC2 or by modifying the active site of these enzymes which can thus no longer catalyze a given substrate. The terms (mTOR) "antagonist" and (mTOR) "inhibitor" are used without preference according to the present technology.

In one aspect, processes are providing for preparing pyrazolopyrimidine analogs. The processes also include the preparation of a number of intermediate compounds useful in the preparation of pyrazolopyrimidine analogs. In one aspect, a process is provided to produce a pharmaceutical compound represented by the general Formula (I), Formula (Ia), Formula (Ib), Formula (Ic), Formula (II), Formula (III) and Formula (IV) in a substantially isomerically pure form. The process is more regioselective than the known synthetic methods, and may be conducted to prepare commercially useful quantities. In another aspect, synthetic methods are provided for producing analogues of pyrazolopyrimidine compounds which are regioselective, efficient, scalable and economical. In another aspect, substantially isomerically pure compounds and intermediates are produced by the above processes.

In one embodiment, the present technology relates to a new process for the preparation of pyrazolopyrimidine analogs using new intermediates. The process is a much more efficient, commercially viable process to manufacture the target compounds. In other embodiments, novel synthetic intermediate compounds useful for the synthesis of pyrazolopyrimidine analogs are provided.

One embodiment provides a process for the preparation of a compound of Formula I, 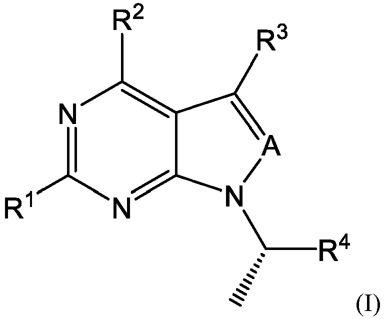 or a hydrate, solvate, or pharmaceutically acceptable salt thereof, which comprises:
reacting a boronic acid compound of Formula II or a salt thereof: 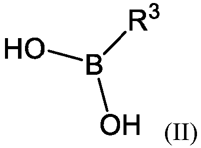
with a compound of Formula III or a salt thereof: 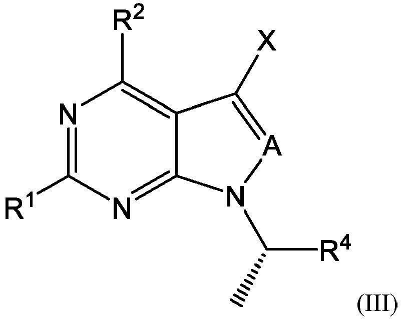
under Suzuki coupling conditions in the presence of a first base to generate a compound of Formula I;
wherein:
   X represents F, Cl, Br or I;
   A represents a -CH radical or a nitrogen atom;
   R¹ represents an -NH₂, -NHMe or -NHEt radical;
   R² represents a hydrogen atom, a halogen atom chosen from F, Cl, Br and I, an -NH₂, -NHalkyl, -NHAc, nitrile, methyl (Me), ethyl (Et), trifluoromethyl, -OH or methoxy radical;
   wherein when R² is other than an -NH₂ radical, then R¹ represents an -NH₂ radical;
   R³ represents a simple or fused bicyclic aromatic or heteroaromatic radical, which is unsubstituted or mono- or polysubstituted with one or more radicals R^{a}; wherein R^{a} is selected from a halogen atom chosen from F, Cl, Br and I, -NH₂, -NHR⁵, nitrile, methyl, ethyl, trifluoromethyl, -OR⁶;
   R⁵ represents hydrogen atom, a radical selected from cyclopropyl, acyl, saturated or unsaturated C₁-C₆ alkyl, optionally interrupted with a heteroatom O or S, and unsubstituted or substituted with a C₃-C₅ cycloalkyl or heterocycloalkyl; and
   R⁶ represents a hydrogen atom or a methyl radical;
   R⁴ represents a hydrogen atom, a linear or branched C₁-C₁₀ alkyl radical, a saturated or unsaturated C₃-C₁₀ ring or bicycle, optionally interrupted with one or more heteroatoms O, S and N, and unsubstituted or substituted with a radical selected from sulfone, fluoro, cyano, ester, -NR⁷, -NR⁷R⁸, C₃-C₆ cycloalkyl or heterocycloalkyl, or an aromatic ring or a heterocycle which is unsubstituted or mono- or polysubstituted with a halogen atom chosen from Cl and F or a radical selected from -OH, -OMe, trifluoromethyl, methyl, ethyl, -NH₂, -NHMe, -NMe₂; and
   R⁷ and R⁸ representing, independently of each other, a hydrogen atom, a C₁-C₃ alkyl, cyclopropyl or acyl radical, or together forming a C₃-C₅ ring; and
   optionally converting compound of Formula I to a hydrate, solvate, , or pharmaceutically acceptable salt thereof.

In certain embodiments, X is Cl. In other embodiments, X is I. In certain embodiments, X is Br. In certain embodiments A is a nitrogen atom. In certain embodiments, R¹ is -NH₂. In certain embodiments, R² is -NH₂.

In certain embodiments, R³ is a fused bicyclic heteroaromatic radical. In certain embodiments, R³ is benzoxazole radical. In certain embodiments, R³ is benzoxazole radical which is mono-substituted with one or more radicals R^{a}. In certain embodiments, R^{a} is -NH₂. In certain embodiments, R³ has the structure:

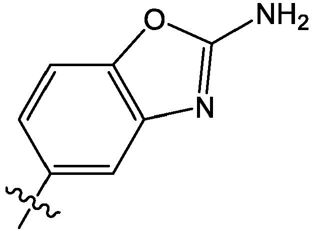

In certain embodiments, R⁴ is a linear or branched C₁-C₁₀ alkyl. In certain embodiments, R⁴ is a linear or branched C₁-C₆ alkyl group. In certain embodiments, R⁴ is an isopropyl or an isobutyl radical. In certain embodiments, R⁴ is an isopropyl radical.

In a preferred embodiment, the compound of Formula I has Formula Ia: 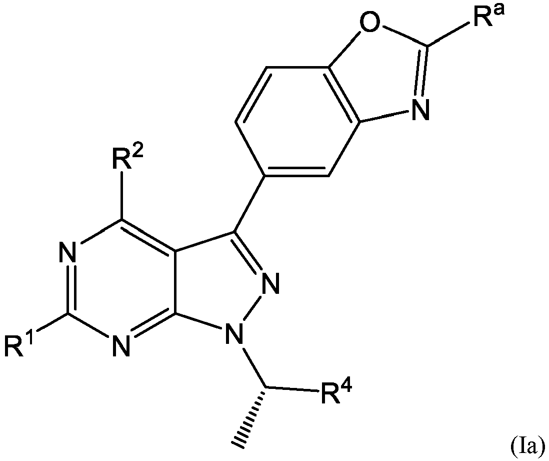
or a hydrate, solvate, or pharmaceutically acceptable salt thereof;
wherein the variables are defined as above.

In a more preferred embodiment, the compound of Formula I has Formula Ib: 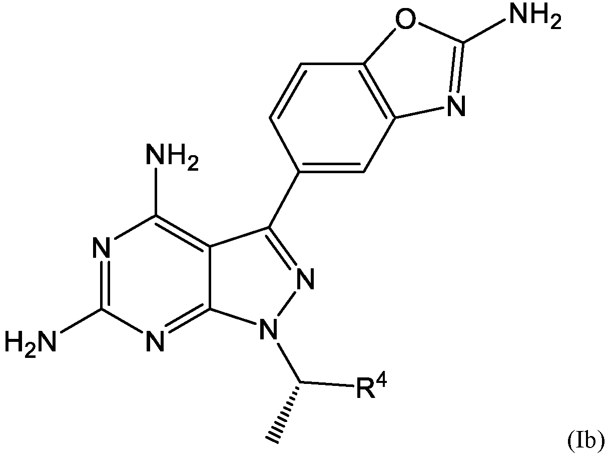
or a hydrate, solvate, or pharmaceutically acceptable salt thereof;
wherein the variables are defined as above.

In a still more preferred embodiment, the compound of Formula I has Formula Ic: 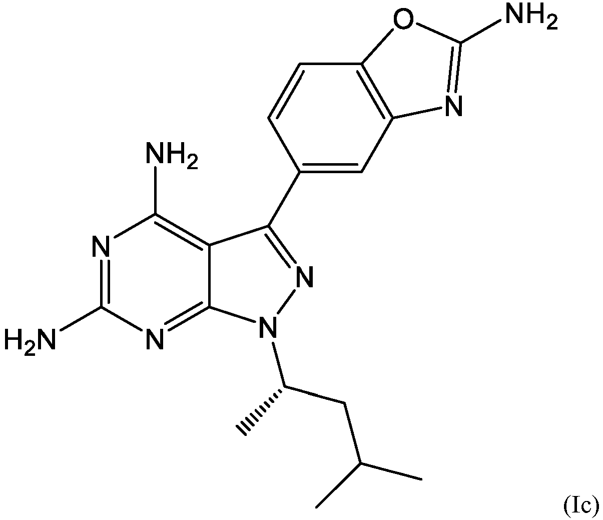 or a hydrate, solvate, or pharmaceutically acceptable salt thereof.

Conditions suitable for a Suzuki coupling reaction of compounds of Formula II and Formula III are well known in the art *(see e.g.,* A. Suzuki, in "Metal-catalyzed Cross-coupling Reactions", F. Diederich and P.J. Stang (Eds.), Wiley- VCH; Weinheim (1998), pp49- 89). In one or more embodiments, the Suzuki coupling conditions may include heating a reaction mixture comprising the compound of Formula II or a salt thereof, the compound of Formula III or a salt thereof, a Suzuki coupling catalyst, a first base and a solvent. The catalyst is suitably formed in situ from a suitable metal salt and a ligand. Suitable catalysts include, but are not limited to, a palladium catalyst such as tetrakis(triphenylphosphine)palladium (Pd(PPh₃)₄), dichlorobis(triphenylphosphine)palladium (PdCl₂(PPh₃)₂), palladium(II)acetate (Pd(OAc)₂), dichlorobis(triethylphosphine)palladium (PdCl₂(PEt₃)₂), [1,1'-bis(diphenylphosphino)ferrocene] dichloropalladium(II) (Pd(dppf)₂Cl₂), dichloro[1,1'-bis(diphenylphosphino)ferrocene]-palladium dichloromethane complex (PdCl₂(dppf).CH₂Cl₂), [1,1'-bis(dicyclohexylphosphino)ferrocene] dichloropalladium, tetrakis(tri(o-tolyl)phosphine)palladium, the combination of palladium(II)acetate and l,l'-bis (diphenylphosphanyl)ferrocene (DPPF) or the related dichloro[l,r-bis(diphenylphosphino) ferrocene]palladium (II) dichloromethane adduct, or the combination of palladium(II) acetate" and tricyclohexylphospliine (Cy₃P), or the related bis(tricyclohexylphosphine)-palladium (0) or trans-dichlorobis(tricyclohexylphosphine) palladium (II). In one or more embodiments embodiment, the palladium catalyst is selected from Pd/C, PdCl₂(PPh₃)₂, Pd(PPh₃)₄, and PdCl₂(dppf)₂, Pd(OAc)₂, Pd₂(dba)₃, Palladacycles (complexes containing at least one carbon-palladium bond) or a combination of any two or more thereof. In one or more embodiments, the palladium catalyst is Pd(PPh₃)₄.

Suitable solvents for the Suzuki coupling reaction include, but are not limited to, an alcohol, *e.g.,* methanol (MeOH), ethanol (EtOH), isopropyl alcohol (iPrOH), 1-propanol, 1-butanol, 2-butanol, a ketone, *e.g.,* acetone, methyl ethyl ketone (MEK), methyl isobutyl ketone, a hydrocarbon, *e.g.,* toluene, xylene, hexanes, heptanes, cyclohexane, a halogenated hydrocarbon, *e.g.,* dichloromethane (DCM), ethylene dichloride, chloroform, an ester, *e.g.,* ethyl acetate (EtOAc), n-propyl acetate, n-butyl acetate, t-butyl acetate, an ether, *e.g.,* diethyl ether, diisopropyl ether, methyl t-butyl ether, tetrahydrofuran (THF), 2-methyltetrahydrofuran (MeTHF), dioxane, a polar aprotic solvent, *e.g.,* N,N-dimethylformamide (DMF), N,N-dimethylacetamide (DMA), dimethylsulfoxide, sulfolane, N-methylpyrrolidone, a nitrile, *e.g.,* acetonitrile (CAN), propionitrile, halogenated solvents, *e.g.,* benzotrifluoride, water; or mixtures thereof. In one or more embodiments, the solvent may include water and a protic solvent selected from the group consisting of isopropyl alcohol, methanol, ethanol, propanol, butanol, tert-butanol. In one or more embodiments, the solvent is water. In one or more embodiments, the solvent is isopropyl alcohol. In one or more embodiments, the solvent includes water and isopropyl alcohol.

Suitable bases for the Suzuki coupling reaction, *i.e.,* the first base, may include both organic and inorganic bases. Examples of suitable inorganic bases include, but are not limited to, potassium carbonate (K₂CO₃), sodium carbonate (Na₂CO₃), potassium bicarbonate (KHCO₃), tripotassium phosphate (K₃PO₄), trisodium phosphate (Na₃PO₄), dipotassium hydrogen phosphate (K₂HPO₄), disodium hydrogen phosphate (Na₂HPO₄), or a combination of any two or more thereof. Examples of suitable inorganic bases include, triethylamine (TEA), tributylamine (TBA), 1,8-Diazabicycloundec-7-ene (DBU), ,5-diazabicyclo[4.3.0]non-5-ene (DBN), 1,5,7-triazabicyclo[4.4.0]dec-5-ene (TBD), 7-methyl-1,5,7-triazabicyclo[4.4.0]dec-5-ene (MTBD), 1,1,3,3-tetramethylguanidine (TMG), 4-dimethylaminopyridine (DMAP), 1,4-diazabicyclo[2.2.2]octane (DABCO), and the like or a combination of any two or more thereof. In one or more embodiments, the first base includes potassium carbonate. In one or more embodiments, the first base includes tripotassium phosphate.

Suitable Suzuki coupling methods are known in the art. For example, a solvent solution of isopropyl alcohol and water is degassed by bubbling N₂ gas. The bicyclic compound of Formula III or a salt thereof along with a first base such as tripotassium phosphate and a catalyst such as tetrakis(triphenylphosphine)palladium is added to the degassed solvent solution followed by a portion of the boronic acid compound of Formula II or a salt thereof. The solution is warmed to a suitable temperature and maintained at that temperature for a suitable period of time with stirring. The remaining portion of the boronic acid compound of Formula II is then added to minimize homocoupling impurity. The reaction mixture is then stirred at a suitable temperature for a suitable period of time to achieve the desired conversion to a compound of Formula I.

The Suzuki reaction can be conducted at a temperature of about ambient temperature to about 150 °C, including without limitation, from about 40 °C to 140 °C, from about 50 °C to 130 °C, from about 60 °C to 120 °C, from about 65 °C to 110 °C, from about 70 °C to 100 °C, or from about 75 °C to 90 °C, or any range including and/or in-between any two of these values. Suitable reaction times depend on the temperature and other conditions, and can be about 8 to 36 h, including, without limitation, from about 10 h to about 24 h, from about 12 h to about 20 h, from about 15 h to about 20 h, or from about 16 h to about 19 h or any range including and/or in-between any two of these values.

The compound of Formula I may be isolated, purified and crystallized using suitable methods known in the art. Suitable isolation, purification and crystallization conditions are such that the impurities are purged efficiently, the desired polymorphic form is obtained in a robust manner and that residual solvent levels in the isolated material are below required limits. For example, the compound of Formula I may be dissolved in a suitable solvent such as 2-MeTHF or acetone, optionally followed by solvent swap to EtOAc or acetone. Residual solvent can be removed using high vacuum and/or high temperature. Polymorph study was carried by: 1) determination of the solubility of the compound, e.g., Compound A in range of solvents; 2) short slurry ripening process by stirring at 16 h at r.t.; 3) evaporation of dryness; 4) recrystallization and slurry ripening with 4 solvents; 5) long slurry ripening over 10 d at r.t. to test stability of the polymorph in other solvents.

The bicyclic compound of Formula III used in the above reaction can be obtained by alkylating a compound of Formula IV as shown in the scheme below. 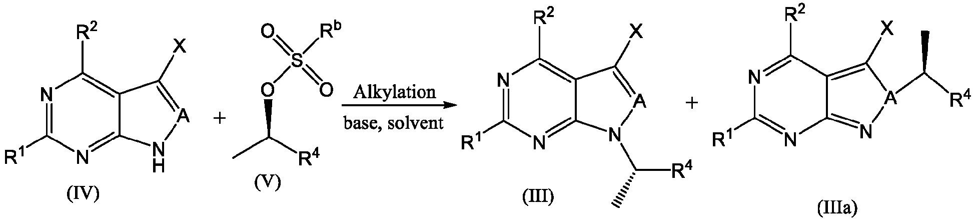 wherein the variables are as defined above.

For the regioselective alkylation reaction, a suspension of bicyclic halo compound of Formula IV is warmed with a second base in a suitable solvent to a suitable temperature to which a solution of the alkylsulfonate, compound of Formula V is added. The reaction mixture is then stirred at a suitable temperature for a suitable period of time to achieve the desired conversion to a compound of Formula III or a mixture of III and IIIa.

Suitable bases for the alkylation reaction, *i.e.* the second base, include, but are not limited to, metal carbonates, such as sodium carbonate, cesium carbonate, potassium carbonate; rubidium carbonate; divalent metal carbonates such as calcium carbonate, magnesium carbonate, strontium carbonate, barium carbonate, manganese carbonate, and the like, metal bicarbonates, such as sodium bicarbonate, cesium bicarbonate, potassium bicarbonate; metal hydrides, such as sodium hydride, potassium hydride, cesium hydride; metal hydroxides, such as sodium hydroxide, potassium hydroxide cesium hydroxide; metal alkoxides, such as sodium methoxide and potassium t-butoxide; and combinations of any two or more thereof. In one or more embodiments, the second base includes bases composed of the heavy alkali metals, such as cesium bases *e.g.,* cesium carbonate, cesium bicarbonate, cesium hydroxide potassium carbonate, cesium hydride, potassium phosphate tribasic, dibasic and monobasic, organic bases including but not limited to tetramethylguanidine, 1,5-Diazabicyclo[4.3.0]non-5-ene (DBN), 8-diazabicyclo[5.4.0]undec-7-ene (DBU), triazabicyclodecene (TBD), 7-methyl-1,5,7-triazabicyclo (4.4.0)dec-5-ene (MTBD), phosphazene bases including but not limited to P1, BEMP, P2 and P4, or a combination of any two or more thereof. In one or more embodiments, the second base includes cesium carbonate.

Both the bicyclic halo compound of Formula IV and the alkylating agent of Formula V may be dissolved in a suitable solvent prior to alkylation. Suitable solvents for the alkylation reaction include, but are not limited to, anhydrous and aprotic solvents such as dimethyl sulfoxide (DMSO), dimethylformamide (DMF), dimethylacetamide (DMAC/DMA), N-Methyl-2-pyrrolidone (NMP), tetramethylene sulfone (sulfolane), butadiene sulfone (3-sulfolene), dimethyl sulfone, nitromethane, propylene carbonate, acetonitrile, ether solvents including but not limited to 2-methyltetrahydrofuran (MeTHF), tetrahydrofuran (THF), diethyl ether, diisopropyl ether (DIPE), 1,4-dioxane, dimethoxyethane (DME), dipropylene glycol or a combination of any two or more thereof. In one or more embodiments, the solvent is dimethyl sulfoxide.

Compounds suitable as alkylating agents include the halides, and esters such as sulfates and sulfonates. In one or more embodiments the alkylating agent is a sulfonate compound of Formula V, wherein Rb represents a hydrogen atom, a linear or branched C1-C10 alkyl radical or a C1-C6 aryl radical. In one or more embodiments, Rb is methyl or ethyl. In one or more embodiments, the alkylating agent is a sulfonate, such as for example, trifluoromethanesulfonate, methanesulfonate, ethanesulfonate, benzenesulfonate and p-toluenesulfonate, or a salt thereof.

The alkylation reaction can be conducted at a temperature of about 10 °C to 100 °C, including without limitation, from about 15 °C to 80 °C, from about 20 °C to 70 °C, from about 30 °C to 60 °C, from about 35 °C to 55 °C, from about 30 °C to 50 °C, or from about 40 °C to 50 °C, or any range including and/or in-between any two of these values. Suitable reaction times depend on the temperature and other conditions, and can be about 5 to 36 h, including, without limitation, from about 10 h to about 24 h, from about 12 h to about 20 h, from about 15 h to about 20 h, or from about 16 h to about 20 h or any range including and/or in-between any two of these values.

The reaction conditions for the alkylation reaction are such that regioisomer of Formula III or a salt thereof is generated in a greater amount than the regioisomer of Formula IIIa or a salt thereof. For example, the ratio of regioisomer of Formula III to regioisomer of Formula IIIa is greater than about 60:40, such as for example about 65:35, about 70:30, about 75:25, about 80:20, about 85:15, about 90:10, about 95:5, about 99:1, or about 100:0. In one or more embodiment, the ratio of regioisomer of Formula III to regioisomer of Formula IIIa is greater than about 80:20.

The process may further include reacting the compound of Formula III with an acid in a suitable solvent to generate an acid addition salt of compound of Formula III. For example, the process may include treating a compound of the Formula III and/or Formula IIIa with an acid, selected from the group consisting of oxalic acid, acetic acid, benzoic acid, formic acid, hydrofluoric acid, hydrochloric acid, and the like, in a solvent selected from the group consisting of ethanol, methanol, isopropanol, butanol, cyclopentyl methylester, methyl acetate, ethyl acetate, isopropyl acetate, water and mixtures of two or more thereof, to form the acid salt of a compound of the Formula III and/or Formula IIIa. In one or more embodiments, the acid is oxalic acid and the acid addition salt is the oxalate salt of compound of Formula III. The reaction is conducted at a temperature ranging from 10°C to 90°C, including about 20 °C to 70 °C, about 30 °C to 65 °C, or from about 40 °C to 60 °C. The acid addition salt can be isolated by conventional methods such as filtration, washing and drying.

The alkylating agent of Formula V can be obtained from the corresponding alcohol compound of Formula VI as shown in the scheme below. 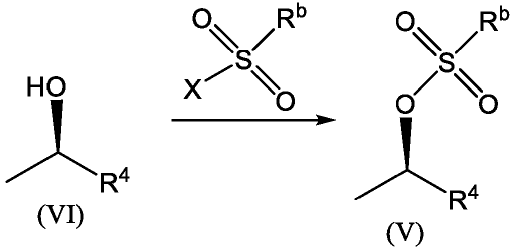 wherein the variables are as defined above.

The alkylating agent of Formula V can be obtained by reacting an a substituted alcohol *(e.g.,* (R)-4-methylpentanol) with a substituted sulfonyl halide *(e.g.,* methanesulfonyl chloride) in an organic solvent in the presence of a tertiary amine, and separating and washing the resulting solvent solution with water and evaporating to dryness.

Suitable solvents include those listed herein for the coupling reaction. Examples of organic solvents employed for the reaction include, but are not limited to DMA, DMF, toluene, DMSO, ACN, THF, DCM, EtOAc, MEK, acetone, and dioxane, or a mixture of any two or more thereof. In one or more embodiments, the solvent is DCM. Suitable tertiary amines include, but are not limited to alkylamines such as trimethylamine, triethylamine, and diisopropylethylamine; dialkylanilines such as dimethylaniline and diethylaniline; and dialkylbenzylamines such as dimethylbenzylamine, or a combination of any two or more thereof. In one or more embodiments, the tertiary amine is triethyl amine.

The boronic acid compound of Formula II or a salt thereof used in the above Suzuki coupling reaction can be obtained by borylation of the corresponding organohalide compound of Formula VII, optionally followed by salt formation as shown in the scheme below. 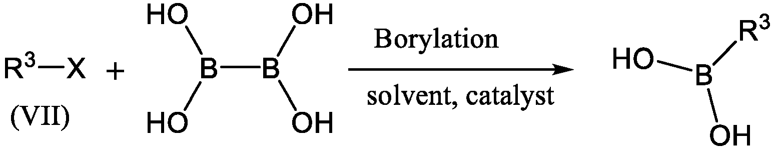 wherein the variables are as defined above.

The organohalide compound of Formula VII, prior to borylation, can be subjected to discoloration or color improvement using activated carbon materials known in the art, such as for example, commercially available DARCO^{™} activated carbon. Suitable alkylation methods are known in the art. For example, the organohalide can be reacted with a tetrahydroxydiboron and diol in the presence of a transition metal catalyst and a third base. The organohalide compound of Formula VII along with a third base is mixed with a solvent and a diol, and the solution is degassed with nitrogen. A suitable transition metal catalyst is added to the solution followed by tetrahydroxydiboron. The reaction mixture is stirred at a suitable temperature for a suitable period of time to obtain the desired conversion to the boronic acid compound of Formula II.

Suitable bases for the borylation reaction, *i.e.,* the third base, include, but are not limited to, potassium acetate, sodium acetate, lithium acetate, potassium phosphate, sodium phosphate, lithium phosphate, potassium carbonate, sodium carbonate, lithium carbonate, trimethylamine or triethylamine. Suitable transition metal catalysts include a Group 8 metal of the Periodic Table, *e.g.* Ni, Pt, Pd or Co. In one or more embodiments, the transition metal catalyst includes one or more phosphine ligands which are complexing the transition metal. Examples of catalysts include Pd or Co compounds like PdCl₂, CoCl₂ and Pd(OAc)₂, palladium phosphine complexes like Pd(PPh₃)₄, PdCl₂(dppf), P(i-Pr)₃, and chloro[(tri-tert-butylphosphine)-2-(2-aminobiphenyl)] palladium(II) *(e.g.,* Pd-168). Suitable diols include, but are not limited to, ethylene glycol, 1,2-propanediol, 1,3-propanediol, 1,3-butanediol, 2-methyl-2,4-pentanediol, pinacol neopentyl glycol, and the like, or a combination of any two or more thereof. Suitable solvents such as those listed herein may be employed for the borylation. In one embodiment, the solvent includes an alcoholic solvent such as methanol, ethanol, isopropanol and the like or combinations thereof. In one embodiment, the solvent is methanol.

The process may further include reacting the compound of Formula II with an acid in a suitable solvent to generate an acid salt of compound of Formula II. For example, the process may include reacting the compound of Formula II with a strong acid. Any acid may be used for this purpose. Exemplary acids include but are not limited to, hydrochloric acid, hydrobromic acid, sulfuric acid, acetic acid, nitric acid and perchloric acid. In one embodiment, the acid is hydrochloric acid. In one or more embodiments, the solvent includes an alcoholic solvent such as methanol, ethanol, isopropyl alcohol and the like or combinations thereof. In one embodiment, the solvent is isopropyl alcohol.

In one embodiment, the process for preparing a compound of Formula (I) is as depicted in the following Scheme I.

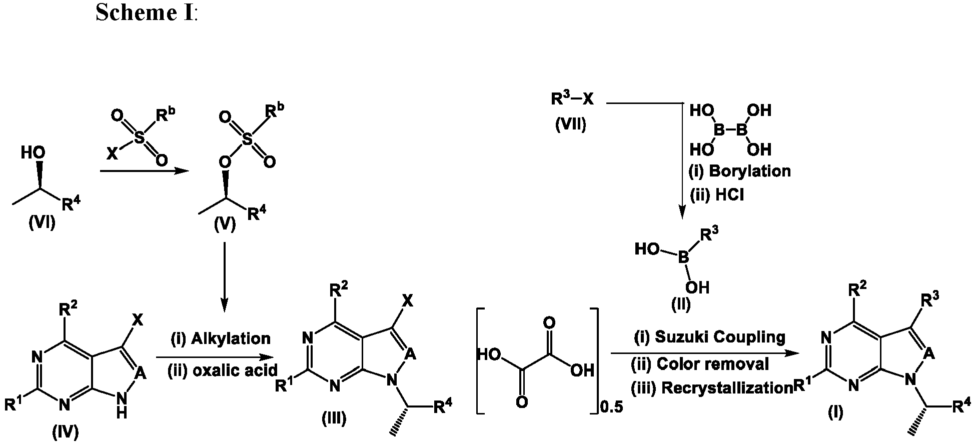

For the reactions described herein, suitable solvents include, but are not limited to, an alcohol, *e.g.,* methanol, ethanol, isopropyl alcohol, 1-propanol, 1-butanol, 2-butanol, a ketone, *e.g.,* acetone, ethyl methyl ketone, methyl isobutyl ketone, a hydrocarbon, *e.g.,* toluene, xylene, hexanes, heptanes, cyclohexane, a halogenated hydrocarbon, *e.g.,* dichloromethane (DCM), ethylene dichloride, chloroform, an ester, *e.g.,* ethyl acetate, n-propyl acetate, n-butyl acetate, t-butyl acetate, an ether, *e.g.,* diethyl ether, diisopropyl ether, methyl t-butyl ether, tetrahydrofuran (THF), dioxane, a polar aprotic solvent, *e.g.,* N,N-dimethylformamide, N,N-dimethylacetamide, dimethylsulfoxide, sulfolane, N-methylpyrrolidone, a nitrile, *e.g.,* acetonitrile, propionitrile, water; or mixtures thereof.

In general, for the reactions described herein, suitable temperatures for the reaction are less than about 500° C, less than about 300° C, less than about 200° C, less than about 100° C, less than about 80° C, less than about 60° C, less than about 40° C, less than about 20° C, less than about 0° C, less than about -10° C, or any other suitable temperatures. In some embodiments, the reaction temperature is the room temperature. Suitable reaction times depend on the temperature and other conditions, and may be less than about 60 hours, less than about 40 hours, less than about 30 hours, less than about 20 hours, less than about 10 hours, less than about 5 hours, less than about 2 hours, less than about 1 hour, or any other suitable times. Longer times may also suitable.

In certain embodiments, starting from the (R)-4-methylpentan-2-ol and 5-bromobenzo[d]oxazol-2-amine, Compound A can be prepared by the following scheme:

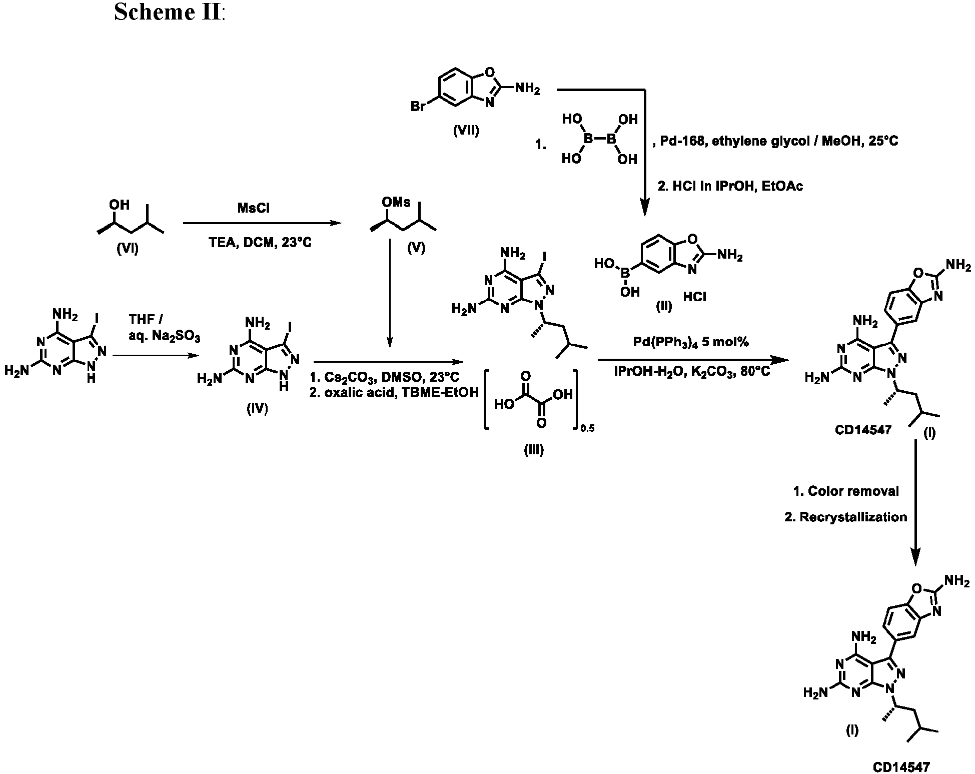

As depicted in Scheme II, (R)-4-methylpentanol (II) is transformed to the corresponding mesylate by reacting it with methanesulfonyl chloride in presence of a base such as triethylamine to form (R)-4-methylpentan-2-yl methanesulfonate (V), which is then used for stereoselectively alkylating 3-iodo-1H-pyrazolo[3,4- d]pyrimidine-4,6-diamine (IV), in presence of an cesium base such as Cs₂CO₃, to provide the alkylated regioisomer product, which is then reacted with a solution of oxalic acid in ethanol to obtain (S)-3-iodo-1-(4-methylpentan-2-yl)-1H-pyrazolo[3,4-d]pyrimidine-4,6- diamine oxalate (III). The oxalate compound (III) is subjected to a Suzuki coupling reaction by portionwise addition of (2-aminobenzo[d]oxazol-5-yl)boronic acid hydrochloride in the presence of aa Suzuki coupling catalyst such as Pd(PPh₃)₄, a base such as tripotassium phosphate and a solvent such as isopropyl alcohol and heating the mixture to obtain crude (S)-3-(2-aminobenzo[d]oxazol-5-yl)-1-(4-methylpentan-2-yl)-1H-pyrazolo[3,4-d] pyrimidine -4,6-diamine. The crude compound can be subjected to color removal and recrystallization to obtain pure compound (I) in high yield and of high purity.

One embodiment of the present technology relates to a novel synthetic intermediate oxalic acid salt represented by the following structural formula (IIIb) and methods of preparing them. 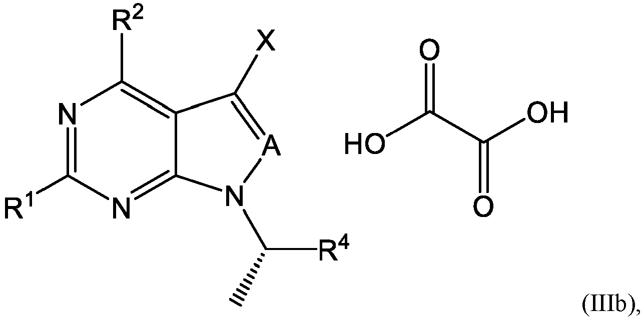 wherein:
X represents F, Cl, Br or I;
A represents a -CH radical or a nitrogen atom;
R¹ represents an -NH₂, -NHMe or -NHEt radical;
R² represents a hydrogen atom, a halogen atom chosen from F, Cl, Br and I, an -NH₂, -NHalkyl, -NHAc, nitrile, methyl (Me), ethyl (Et), trifluoromethyl, -OH or methoxy radical;
wherein when R ₂ is other than an -NH ₂ radical, then R ₁ represents an -NH ₂ radical;
R⁴ represents a hydrogen atom, a linear or branched C₁-C₁₀ alkyl radical, a saturated or unsaturated C₃-C₁₀ ring or bicycle, optionally interrupted with one or more heteroatoms O, S and N, and unsubstituted or substituted with a radical selected from sulfone, fluoro, cyano, ester, -NR⁷, -NR⁷R⁸, C₃-C₆ cycloalkyl or heterocycloalkyl, or an aromatic ring or a heterocycle which is unsubstituted or mono- or polysubstituted with a halogen atom chosen from Cl and F or a radical selected from -OH, -OMe, trifluoromethyl, methyl, ethyl, -NH₂, -NHMe, -NMe₂; and
R⁷ and R⁸ representing, independently of each other, a hydrogen atom, a C₁-C₃ alkyl, cyclopropyl or acyl radical, or together forming a C₃-C₅ ring.

In some embodiments, X is Br, Cl or I. In some embodiments, R¹ is -NH₂. In some embodiments, R² is -NH₂. In some embodiments, R⁴ is a linear or branched C₁-C₆ alkyl radical.

In some embodiments, the compound of formula (III) has the formula (IIIc)

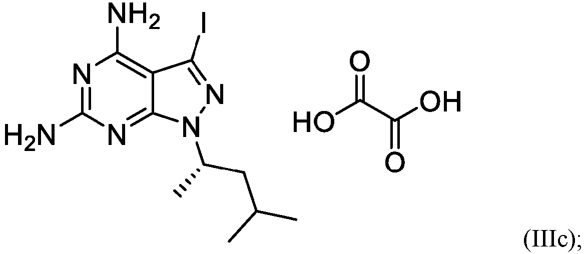

Another embodiment of the invention relates to novel synthetic boronic acid intermediates represented by the following structural formula (IIa) and methods of preparing them. 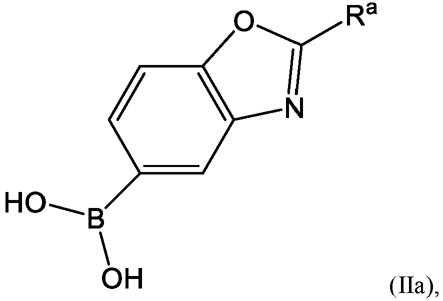 wherein:
R^{a} represents a halogen atom chosen from F, Cl, Br and I, -NH₂, -NHR⁵, nitrile, methyl, ethyl, trifluoromethyl, or -OR⁶;
R⁵ represents hydrogen atom, a radical selected from cyclopropyl, acyl, saturated or unsaturated C₁-C₆ alkyl, optionally interrupted with a heteroatom O or S, and unsubstituted or substituted with a C₃-C₅ cycloalkyl or heterocycloalkyl; and
R⁶ represents a hydrogen atom or a methyl radical.

In some embodiments, R^{a} is -NH₂, -NHR⁵, wherein R⁵ is C₁-C₆ alkyl. In some embodiments, R^{a} is -NH₂.

In some embodiments, the compound of formula (II) has the formula (IIb):

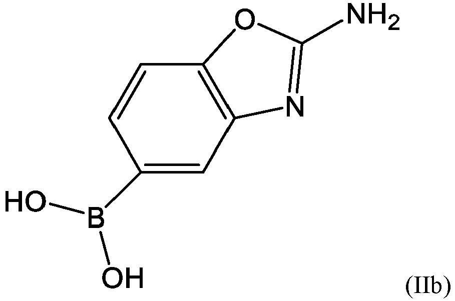

In one or more embodiments, the compound of Formula III prepared according to the process described herein has at least 70%, 80%, 90%, 95%, 97%, 98%, 99%, 99.5%, 99.9% or 100% chiral purity. In one or more embodiments, the compound of Formula I prepared according to the process described herein has at least 70%, 80%, 90%, 95%, 97%, 98%, 99%, 99.5%, 99.9% or 100% chiral purity. In one or more embodiments, the compound of Formula I prepared according to the process described herein has greater than 95% chiral purity. In one or more embodiments, the compound of Formula I prepared according to the process described herein has greater than 98% chiral purity. In one or more embodiments, the compound of Formula I prepared according to the process described herein has greater than 99% chiral purity. The chiral compounds of the present technology are obtained in enantiomerically pure form, for instance with an enantiomeric excess (ee) > 90%, > 95%, > 98%, or > 99%.

The present technology advantageously provides an easy and fast separation method for the chiral isomers of pyrazolopyrimidine based mTOR inhibitor compounds and intermediate compounds such as the compound of Formula III and provides enantiomerically enriched products.

The compounds prepared by the methods described herein can be converted in to its pharmaceutically acceptable salts using the methods known in the art. The salts can be produced before or after the isolation of the particular compound. Thus in one embodiment, pharmaceutically acceptable salts of compound of Formula I, Ia, Ib or Ic can be prepared.

The present technology also relates to pharmaceutically acceptable derivatives, including salts and pro-drugs, tautomers, metabolites, solvates, and hydrates of the compounds described herein. The compounds of Formula I, Ia, Ib and Ic may exist as solvates, especially hydrates. Hydrates may form during manufacture of the compounds or compositions comprising the compounds, or hydrates may form over time due to their hygroscopic nature. Compounds of Formula I, Ia, Ib and Ic may exist as organic solvates as well, including DMF, ether, and alcohol solvates among others. The identification and preparation of any particular solvate is within the skill of the ordinary artisan of synthetic organic or medicinal chemistry.

The invention also includes pharmaceutical compositions comprising a compound of Formula I prepared by the methods described herein. A pharmaceutical composition may comprise a compound of Formula I, Ia, Ib or Ic and a pharmaceutically acceptable carrier or excipient. As used in the present invention, the term "patient" denotes a mammal and includes human and animal individuals, preferably a human. The term "pharmaceutical" when used herein as an adjective means substantially non-deleterious to the recipient mammal. By "pharmaceutical composition" it is meant the carrier, diluent, excipients and active ingredient(s) must be compatible with the other ingredients of the formulation, and not deleterious to the recipient thereof. A pharmaceutically acceptable carrier includes such carriers as, for example, aqueous solutions, non-toxic excipients including salts, preservatives, buffers and the like, which are described in Remington's Pharmaceutical Sciences, 15th Ed. Easton: Mack Publishing Co., pp. 1405-1412 and 1461-1487 (1975) and The National Formulary XIV., 14th Ed. Washington: American Pharmaceutical Association (1975). The compounds prepared by the methods described herein can be formulated prior to administration. The selection of the formulation should be decided by the attending physician taking into consideration the same factors involved with determining the effective amount.

An aspect of the present invention is also a composition comprising, in a pharmaceutically acceptable medium, a compound of Formula I, Ia, Ib or Ic as defined above or a pharmaceutically acceptable salt thereof. A pharmaceutically acceptable medium denotes a medium that is compatible with and suitable for use in contact with human and animal cells, in particular with the skin, mucous membranes and/or the integuments, without undue toxicity, irritation or allergic response or the like, and commensurate with a reasonable benefit/risk ratio. A pharmaceutically acceptable medium according to the invention may comprise any known adjuvant used in the pharmaceutical field, which is compatible with the mTOR-inhibiting compounds according to the present technology. Suitable examples of pharmaceutically acceptable medium include, but are not limited to solvents, buffers, aromatizing agents, binders, chelating agents, surfactants, thickeners, lubricants, gellants, humectants, moisturizers, preserving agents, antioxidants, calmative agents, pro-penetrating agents, colorants, fragrances and the like, or a mixture thereof. Other optional components, known to a person skilled in the art can be added to the pharmaceutical composition such that the advantageous properties intrinsically associated with the present invention are not, or are not substantially, adversely affected by the envisaged addition at a concentration so that they not harm the advantageous properties of the compounds.

The pharmaceutical compositions of the present technology may be in liquid, solid or gas form and may be administered orally, rectally, topically or parenterally (subcutaneously, intramuscularly or intravenously). In one or more embodiments, the pharmaceutical compositions are administered topically. For administration via oral route, the composition may be in the form of tablets, gel capsules, coated tablets, syrups, suspensions, solutions, powders, granules, emulsions, suspensions of microspheres or nanospheres or lipid or polymeric vesicles allowing controlled release. For administration via parenteral route, the composition may be in the form of solutions or suspensions for perfusion or for injection. For administration via the topical route, the compositions, which are thus more particularly intended for treating the skin and mucous membranes, may be in liquid, pasty or solid form, and more particularly in the form of ointments, creams, milks, pomades, powders, impregnated pads, syndets, solutions, gels, sprays, mousses, lotions, suspensions, sticks, shampoos or washing bases. They may also be in the form of suspensions of microspheres or nanospheres or lipid or polymeric vesicles, or of polymeric or gelled patches, or of hydrogels allowing controlled release of the active compounds. These topical compositions may moreover be either in anhydrous form or in an aqueous form.

The pharmaceutical composition according to the technology may include between 0.001% and 5% of said compound of Formula I, Ia, Ib or Ic or a pharmaceutically acceptable salt thereof, by weight relative to the total weight of the composition. The amount effectively administered to be used according to the invention depends on the desired therapeutic effect, and may thus vary within a wide range. A person skilled in the art, in particular a medical practitioner, can readily, on the basis of his general knowledge, determine the appropriate amounts. The composition may further include one or more of other active ingredients, such as antibiotics, antibacterials, antivirals, antiparasitic agents, antifungal agents, anesthetics, analgesics, antiallergic agents, retinoids, free-radical scavengers, antipruriginous agents, antihistamines, immunosuppressants, corticosteroids, keratolytic agents, intravenous immunoglobulins, antiangiogenic agents, antiinflammatory agents, and the like, or a combination thereof.

In another aspect, provided are pharmaceutical compositions according to the present technology for use as a medicament, in particular in the treatment of diseases involving an mTOR enzyme with serine-threonine kinase activity in a patient. In one or more embodiments, the pharmaceutical compositions may be administered after one or more symptoms have developed. In one or more embodiments, the pharmaceutical compositions may be administered as a preventive measure, for preventing or stopping the progression of a disease or a disorder. In other embodiments, the pharmaceutical compositions may be administered in the absence of symptoms. For example, the pharmaceutical compositions may be administered to a predisposed individual before the appearance of the symptoms (for example in the light of a history of symptoms and/or of genetic factors or other predisposing factors). The treatment may also be continued after the disappearance of the symptoms, for example to prevent or delay their reappearance.

The compound of Formula I prepared by the methods described herein may be used, in a pharmaceutical composition for treating a number of conditions by administering to a subject, such as a human being in need thereof. For example, the compound of Formula I may be used for treating a conditions, for which mTor inhibitors are known to be effective, such as dermatological complaints and cancer.

In one aspect, the pharmaceutical compositions according to the present technology are particularly intended to be used in the treatment of dermatological complaints associated with a keratinization disorder with a proliferative, inflammatory and/or immunoallergic component. The dermatological complaints associated with a keratinization disorder with a proliferative, inflammatory and/or immunoallergic component comprise keratinization conditions or disorders relating to cell proliferation, notably common acne, comedones, polymorphs, acne rosacea, nodulocystic acne, acne conglobata, senile acne, and secondary acnes such as solar acne, medication-related acne or occupational acne, other keratinization disorders, notably ichthyosis, ichthyosiform conditions, Darier's disease, palmoplantar keratoderma, leukoplakia and leukoplakiform conditions, and cutaneous or mucous (buccal) lichen, other dermatological complaints associated with a keratinization disorder with an inflammatory and/or immunoallergic component, notably all forms of psoriasis, whether it is cutaneous, mucous or ungual psoriasis, and even psoriatic rheumatism, or cutaneous atopy, such as atopic dermatitis (or atopic eczema) or respiratory atopy or gingival hypertrophy, all dermal or epidermal proliferations, whether benign or malignant, and whether of viral origin or otherwise, such as common warts, flat warts and verruciform epidermodysplasia, oral or florid papillomatoses, and lesions or proliferations that may be induced by ultraviolet radiation, notably in the case of actinic keratoses, and basal cell and spinal cell epithelioma. In one or more embodiments, the pharmaceutical compositions are intended to be used in the treatment of dermatological complaints associated with a keratinization disorder with a proliferative, inflammatory and/or immunoallergic component, such as psoriasis, atopic dermatitis, actinic keratosis or acne, even more preferentially atopic dermatitis. In one or more embodiments, the pharmaceutical compositions are intended to be used in the treatment of the inflammatory component of atopic dermatitis, and preferentially the topical treatment of the inflammatory component of atopic dermatitis. The term "inflammatory component of atopic dermatitis" means an inflammation involving the CD4+ lymphocytes, eosinophils, mastocytes and Th2 cytokines. Suitable conditions, for which mTor inhibitor is known to be effective, include but not limited to including cancer treatment, such as breast cancer, lung cancer, non-small-cell lung cancer, kidney cancer, renal carcinoma, prostate cancer, blood cancer, liver cancer, ovarian cancer, thyroid cancer, endometrial cancer, lymphoma, renal cell carcinoma, or mantle cell lymphoma.

The present technology relates to novel mTOR-inhibiting compounds of Formula I, Ia, Ib or Ic. Thus, one aspect of the present disclosure is the compounds of Formula I, Ia, Ib or Ic as described above which are intended to be used as medicaments. An aspect of the disclosure is also a composition according to the invention for its use as a medicament, in particular in the treatment of diseases involving an mTOR enzyme with serine-threonine kinase activity in a patient.

It is to be understood that while the invention has been described in conjunction with the above embodiments, that the foregoing description and examples are intended to illustrate and not limit the scope of the invention. Other aspects, advantages and modifications within the scope of the invention will be apparent to those skilled in the art to which the invention pertains.

The invention is further illustrated by, though in no way limited to, the following examples.

### EXAMPLES

### Example 1. Characterization methods

In the process described below, the reaction products were isolated and characterized using liquid chromatography/mass spectrometry (LC/MS), X-ray Powder Diffraction (XPRD), Nuclear magnetic resonance (¹H NMR and ¹³C NMR). Chiral supercritical fluid chromatography (SFC) (ChiralPak IC column) was used to separate and analyze the mixture of regioisomers in order to obtain enantiomers.

### (A) LC/MS

LC/MS were recorded using different methods and apparatus as described below:

**Method A** was recorded on Waters UPLC Acquity system using the following parameters:
Column: Acquity BEH C18 1.7µm 2.1*150mm;
5 min runs
Photodiode array detection from 210 to 400 nm
Solvent system:
- Mobile phase A: Ammonium carbonate aqueous solution 2g/L
- Mobile phase B: MeCN

Gradient:

| **Time (min)** | **Flow (ml/min)** | **%A** | **%B** |
|---|---|---|---|
| 0 | 0.50 | 95 | 5 |
| 0.20 | 0.50 | 95 | 5 |
| 3.30 | 0.50 | 5 | 95 |
| 4.50 | 0.50 | 5 | 95 |
| 4.70 | 0.50 | 95 | 5 |
| 5.00 | 0.50 | 95 | 5 |

**Method B** was recorded on Waters UPLC Acquity system using the following parameters:
Column: Acquity BEH C18 1.7µm 2.1*150mm;
15 min runs
Photodiode array detection from 210 to 400 nm
Solvent system:
- Mobile phase A: Ammonium carbonate aqueous solution 2g/L
- Mobile phase B: MeCN

Gradient:

| **Time (min)** | **Flow (ml/min)** | **%A** | **%B** |
|---|---|---|---|
| 0 | 0.35 | 95 | 5 |
| 1.0 | 0.35 | 95 | 5 |
| 13.0 | 0.35 | 5 | 95 |
| 14.0 | 0.35 | 5 | 95 |
| 14.1 | 0.35 | 95 | 5 |
| 15.0 | 0.35 | 95 | 5 |

**Method C** was recorded on Waters UPLC Acquity system using the following parameters:
Column: Acquity CSH C18 1.7µm 2.1*150mm;
30 min runs
Photodiode array detection from 210 to 400 nm
Solvent system:
- Mobile phase A: Water + 0.1% Formic Acid
- Mobile phase B: Acetonitrile + 0.1% Formic Acid

Gradient:

| **Time (min)** | **Flow (ml/min)** | **%A** | **%B** |
|---|---|---|---|
| 0 | 0.35 | 95 | 5 |
| 1.0 | 0.35 | 95 | 5 |
| 25 | 0.35 | 5 | 95 |
| 28.0 | 0.35 | 5 | 95 |
| 28.1 | 0.35 | 95 | 5 |
| 30.0 | 0.35 | 95 | 5 |

### (B) Chiral Chromatography

Chiral SFC chromatography was performed with a Waters UPC2 coupled with a Diode Array and QDa detector.

**Chiral method D** (chiral analysis of n-1 intermediate synthesis; 3-iodo-1-(4-methylpentan-2-yl)-1H-pyrazolo[3,4-d]pyrimidine-4,6- diamine oxalate):
Column: Chiralpak IG 100*3 mm, 3µm
Flow rate: 2 ml/mn
Sample concentration: 1 mg/mL (Methanol)
Temperature oven: 40 °C
Detection: PDA detector acquisition (220-400 nm)
Injection volume: 1µL
ABRP pressure: 2000 psi
Gradient: Isocratic CO₂ (94%) / Methanol (6%)
Run time: 15 min

### Chiral method E (chiral analysis of Compound A):

Column: Chiralpak IC 100*3 mm, 3µm
Flow rate: 2 ml/mn
Sample concentration: 1 mg/mL (Methanol)
Temperature oven: 40 °C
Detection: PDA detector acquisition (220-400 nm)
Injection volume: 1 µL
ABRP pressure: 2000 psi
Gradient: Isocratic CO₂ (78%) / Methanol +0.1% diethylamine (22%)
Run time: 15 min.

### (C) ¹H NMR and ¹³C NMR

¹H NMR and ¹³C NMR were recorded on a Bruker Avance 400 MHz, 100 MHz. Chemical shifts are expressed in parts per million (ppm) downfield from residual solvent peaks, and coupling constants are reported in hertz (Hz). XRPD analyses were performed on a Bruker D2 Phaser apparatus with the following parameters:

| | |
|---|---|
| **Start End** :5.000 à | **scan type :** TwoTheta/Theta |
| **Step :** 0.014° | **Time:** 0.35 s |
| **Time per Step:** 56.00 s | **Steps:** 3184 |
| **Analysis time :** 1234 s | **PSD opening** : 4.847 |
| **Anode** : Cuivre | **X Ray wavelength** : 1.54184 Å |
| **Temperature** : 25°C | **Rotation** : 5°/min |
| **Goniometer radius** : 141 | **Generator kV :** 30 kV |
| **Generator mA** : 10mA | **Divergence slit :** 0.6 mm |
| **Axial Soller Slit :** 2.5° | **Antiscatter slit :** 8 mm |
| **K β filter** : Ni 0.5 | **Detector** : SSD160 (1D) |

### Example 2. Preparation of (S)-3-(2-aminobenzo[d]oxazol-5-yl)-1-(4-methylpentan-2-yl)-1H-pyrazolo[3,4-d]pyrimidine-4,6-diamine (Compound A)

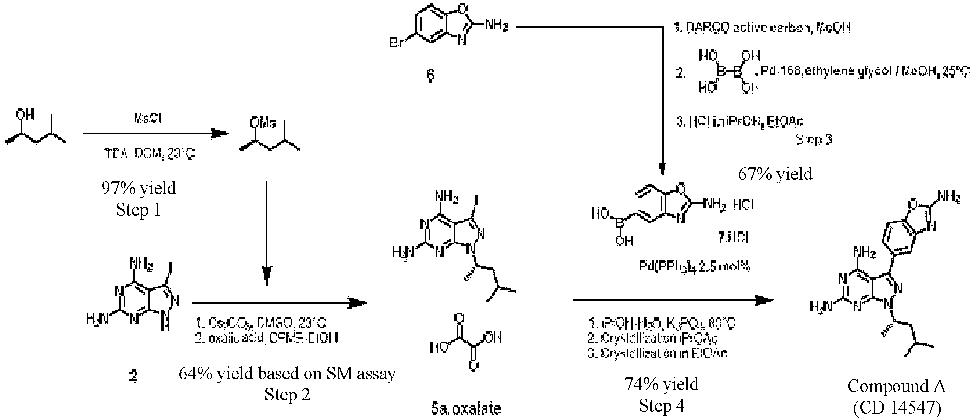

### Step 1: Preparation of 3-iodo-1H-pyrazolo[3,4-d]pyrimidine-4,6-diamine (2)

The pyrazolopyrimidine diamine intermediate, *i.e.*, 3-iodo-1H-pyrazolo[3,4-d]pyrimidine-4,6-diamine (2) can be synthesized as follows.

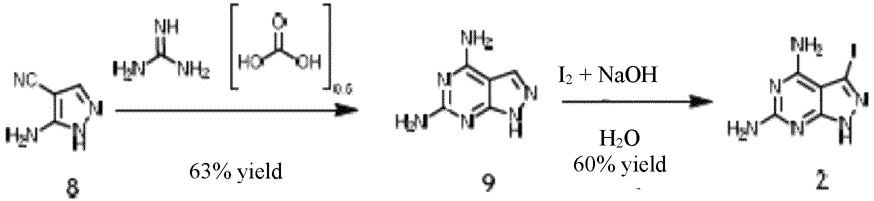

### Step 2: Mesylation of (R)-4-methylpentanol

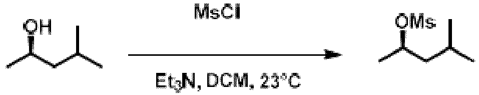

| **Compound** | **MW** | **Mol** | **Eq(mol)** | **Eq(vol )** | **Mass** | **density** | **Vol** |
|---|---|---|---|---|---|---|---|
| (R)-4-methylpentanol | 102.18 | 5.87 | 1.0 | | 600.0 g | | |
| Methanesulfonyl chloride | 114.54 | 5.87 | 1.0 | | 672.6 g | 1.48 | 454.5 mL |
| Triethylamine | 101.19 | 5.87 | 1.0 | | 594.2 g | 0.73 | 816.7 mL |
| DCM | | | | 7.0 | | | 4.2 L |

In a 10 L double jacketed reactor, was charged (R)-4-methylpentanol (600.0 g, 5.87 mol, 1.0 eq) followed by 3.7 L of DCM. Under mechanical stirring and nitrogen flow, trimethylamine (816.7 mL, 5.87 mol, 1.0 eq) was added and the solution cooled to 5°C. Then, a solution of methanesulfonyl chloride (454.5 mL, 5.87 mol, 1.0 eq) in 500 mL of DCM was added slowly keeping the internal temperature below 35°C (30 min). At the end of the addition, the reaction mixture was stirred at 23 °C for 2 h.

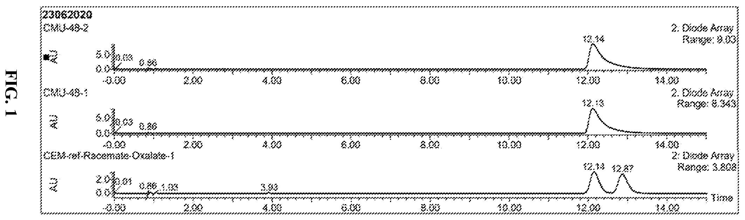

After this time 3.2 L of water were added to the reaction mixture and the biphasic solution was stirred for 15 min. Phases were separated and the organic layer was washed two times with 3.2 L of water, dried over MgSO₄, filtered and evaporated to dryness under reduced pressure to yield CEM-205-001 (1030.0 g, 97%) as a pale yellow liquid. The DMSO-d₆ NMR spectrum confirming the formation of the mesylated product is shown in **FIG. 1****.**

### Step 3: Alkylation of 3-iodo-1H-pyrazolo[3,4-d]pyrimidine-4,6-diamine

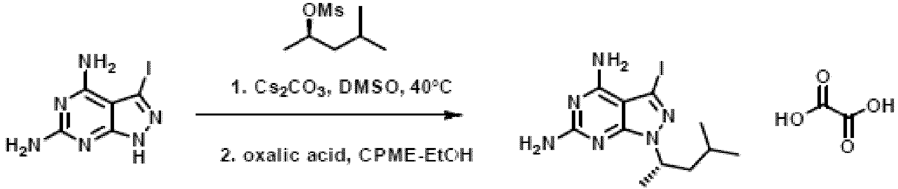

| **Compound** | **MW** | **Purity (w/w)** | **Mol** | **Eq (mol)** | **Eq (vol)** | **Mass** | **density** | **Vol** |
|---|---|---|---|---|---|---|---|---|
| 3-iodo-1H-pyrazolo[3,4-d]pyrimidine-4,6-diamine | 276.04 | 0.82 | 0.95 | 1.0 | | 320.5 g | | |
| (*R*)-4-methylpentan-2-yl methanesulfonate | 180.26 | | 1.74 | 1.8 | | 314.7 g | | |
| Cesium carbonate | 325.80 | | 1.51 | 1.6 | | 492.1 g | | |
| DMSO | | | | | 6.0 | | 1.1 | 2.0 L |
| Oxalic acid | 90.03 | | 0.87 | 0.91 | | 178.4 g | | |

In a 10 L double jacketed glass reactor equipped with mechanical stirring, was charged 3-iodo-1H-pyrazolo[3,4- d]pyrimidine-4,6-diamine (320.5 g, 0.95 mol, 1.0 eq) followed by Cs₂CO₃ (492.1 g, 1.51 mol, 1.6 eq.) and DMSO (2.0 L). This suspension was warmed to 40°C and then a solution of (R)-4-methylpentan-2-yl methanesulfonate (314.7 g, 1.74 mol, 1.8 eq) in DMSO (240 mL) was added. The reaction mixture was stirred at 40°C for 20 h.

**IPC** - An aliquot was taken after 18 h, diluted with MeCN and water and analyzed by HPLC/MS (method A). Complete conversion was observed with a ratio of regioisomers of 75:25.

3.2 L of CPME and 3.2 L of water were added. Phases were separated and the organic layer was washed with water (3.2 L) and then with 0.05 M aqueous HCl solution (2 x 3.2 L).

**LCMS analysis of the organic layer (method A) -** The organic layer was recharged in the reactor and warmed to 60°C. A solution of oxalic acid (78.4 g, 0.87 mol, 0.91 eq) in 320 mL of EtOH was added and the solution was stirred for 30 min at 60°C and then allowed to cool to 23°C over 18 h while a solid crystallized. The solid was collected by filtration, washed with CPME (200 mL) and dried under vacuum at 45°C for 18 h to afford Batch 0048-001 (219.0 g, 51% yield) as an off-white solid. The mother liquors were evaporated to a residual volume of 500 mL and a second crop was collected by filtration, washed with 100 mL of CPME and dried under vacuum at 45°C for 18 h to afford Batch 0048-002 (56.0 g, 13% yield) a an off-white solid.

**Chiral SFC analysis (method D)** - These 2 batches were pooled with other batches obtained under similar conditions at smaller scale and all the solids (total = 585g) were triturated in 3.0 L of CPME for 1h at 23°C and the solid was collected by filtration and dried under vacuum at 45°C for 18h to afford Batch 0052-001 (572.0 g) as a white solid. **Fig. 1** shows the SFC separation and analysis of (S)-3-iodo-1-(4-methylpentan-2-yl)-1H-pyrazolo[3,4-d]pyrimidine-4,6- diamine oxalate.

### Step 4: Borylation of 5-bromobenzoxazol-2-amine

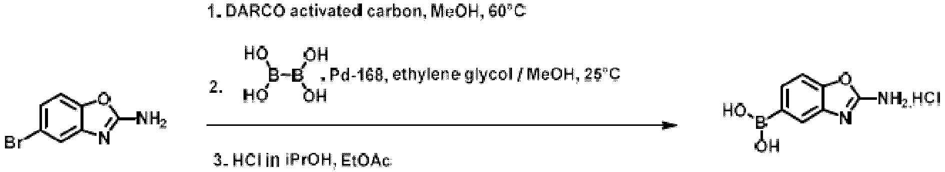

| **Compound** | **MW** | **Purity (w/w)** | **Mol** | **Eq (mol)** | **Eq (vol)** | **Mass** | **Vol** | **Batch/supplier** |
|---|---|---|---|---|---|---|---|---|
| 5-bromobenzoxazol-2-amine | 213.03 | | 1.76 | 1.0 | | 375.0 g | | DLB Pharma |
| DARCO activated carbon (100 mesh) | 12 | | | | | 180.0 g | | |
| Tetrahydroxydiboron | 89.65 | | 2.45 | 1.5 | | 219.7 g | | |
| Potassium acetate | 98.15 | | 4.08 | 2.5 | | 400.8 g | | |
| Pd-168 | 512.40 | | 0.02 | 0.01 | | 8.4 g | | |
| MeOH | | | | | 10.0 | | 3.5 L | |
| Ethylene glycol | | | | | 3.0 | | 1.0 L | |

### (A) SM discoloration:

In a 10 L double jacketed reactor, was charged 5-bromobenzoxazol-2-amine (375.0 g; 1.76 mol; 1.0 eq.) followed by MeOH (6.8 L, 18 vol.). The suspension was warmed to 60°C and turned to a solution. DARCO activated carbon (100 mesh) (180.0 g) was added and the solution was stirred for 1h at 60°C. The solution was then filtered over celite and the orange solution was evaporated to dryness under reduced pressure to afford 5- bromobenzoxazol-2-amine (290.0g, 77%) as a pale beige solid.

### (B) Borylation:

In a 10 L double jacketed reactor under N2 were charged 5-bromobenzoxazol-2-amine (289.0 g; 1.36 mol; 1.0 eq.) and potassium acetate (332.8 g, 3.39 mol, 2.5 eq.) followed by MeOH (2.9 L, 10 vol.) and ethylene glycol (867 mL, 3 vol.). This solution was degassed with nitrogen bubbling for 20 min and then Pd-168 (6.9 g, 0.014 mol, 0.01 eq.) was added at 23°C followed by Tetrahydroxydiboron (180.0 g, 2.03 mol, 1.5 eq.) in 3 portions of 60 g at 23°C over 30 min. Slight off-gassing was observed at each addition with an exotherm and a maximal internal temperature increase to 45°C. After the third addition, the reaction mixture was stirred at 23°C for 1 h.

### IPC - An aliquot was taken after 1 h, diluted with MeOH and analyzed by HPLC/MS (method A)

The reaction mixture was filtered over a pad of celite to remove the catalyst. The filtrate was partially evaporated (residual volume 1.5L) and then water (2.9 L) was added. Precipitation occurred and the suspension was stirred at 50°C for 2 h. The suspension was cooled to 23°C and the solid was collected by filtration and dried under vacuum at 45°C for 18 h to afford a beige solid (232 g, 96.1%).

**HPLC/MS (method A)** - The solid was suspended in iPrOH (2.3 L) and the suspension warmed to 80°C. Then concentrated aq. HCl (118.5 mL of a 11N solution, 1.3 mol, 1.0 eq.) was added and the mixture was stirred at 80°C for 1 h. The reaction mixture was then cooled to 23°C and the solid was isolated by filtration to afford (2-aminobenzo[d]oxazol-5- yl) boronic acid hydrochloride (254g, 87% yield) as an off-white solid.

### Step 5: Suzuki Coupling

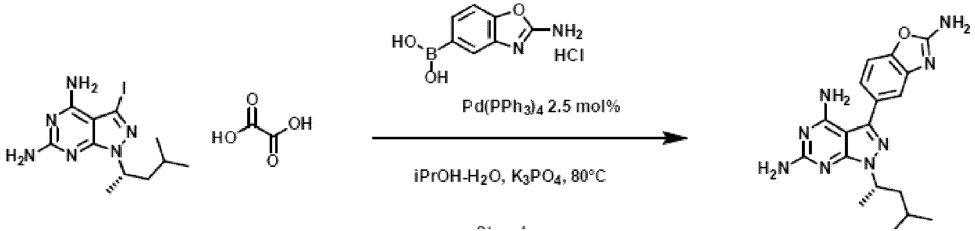

| **Compound** | **MW** | **Mol** | **Eq (mol)** | **Eq (vol)** | **Mass** | **Vol** |
|---|---|---|---|---|---|---|
| (*S*)-3-iodo-1-(4-methylpentan-2-yl)-1H-pyrazolo[3,4-d]pyrimic ine-4,6-diamine oxalate | 450.23 | 0.819 | 1.00 | | 369.00 g | |
| (2-aminobenzo[d]oxazol-5-yl)boronic acid hydrochloride | 214.41 | 0.901 | 1.10 | | 193.2 g | |
| Tripotassium phosphate | 212.26 | 3.29 | 4.00 | | 700.2 g | |
| Pd(PPh3)4 | 1155.59 | 0.02 | 0.025 | | 23.1 g | |
| iPrOH | | | | 10.0 | | 3.7 L |
| water | | | | 6.0 | | 2.2 L |

In a 10 L double jacketed reactor under N2 were charged iPrOH (3.7 L) and water (2.2 L). This solution was degassed by N2 bubbling for 15 min. (S)-3-iodo-1-(4-methylpentan-2-yl)-1H-pyrazolo[3,4-d]pyrimidine-4,6- diamine oxalate (369.00 g; 0.819 mol; 1.00 eq.), tripotassium phosphate (700.2 g, 0.117 mol, 3.50 eq.), tetrakis(triphenylphosphine) palladium (23.1 g; 0.02 mol; 0.025 eq.) were added followed by (2- aminobenzo[d]oxazol-5-yl)boronic acid hydrochloride (105.2 g; 0.49 mol; 0.60 eq.) and the solution was warmed to 85°C and stirred at this temperature for 1 h. The second half of (2-aminobenzo[d]oxazol-5-yl)boronic acid hydrochloride (87.7 g; 0.41 mol; 0.5 eq.) was then added after this time to minimize homocoupling impurity. The reaction mixture was stirred at 85°C for 18 h.

### IPC - An aliquot was taken after 18 h, diluted with MeOH, filtered and analyzed by HPLC/MS (method B)

Complete conversion was observed with small amounts of homocoupling (<0.5%) and debrominated (<2.1%) impurities detected.

The reaction mixture was cooled to 23°C and phases were separated. The organic layer was evaporated to a residual volume of c.a. 700 mL under reduced pressure and 2-MeTHF (3.7 L) was added. The solution was warmed to 40°C and the organic layer was washed with 0.5N aqueous NaOH (2.2 L), then 3 times with an aqueous solution of 5.9g of APDTC in 2.2 L of water and finally with water (1.1 L).

To the organic layer was added DARCO 100 mesh activated carbon (148 g) and the suspension was stirred for 1 hour at 50°C. The suspension was filtered over celite to remove active carbon affording a pale green solution. 2- MeTHF was stripped by iPrOAc to reach a final volume of 3.7 L of iPrOAc.

At this point, 2 extra batches of product obtained from smaller scale test experiments were added for homogenization and seeding:
Batch 0054-1; 30.1 g
Batch 0055-1; 16.2g

The suspension was warmed to 45°C and was stirred at this temperature for 18 h. The suspension was cooled to 23°C, the solid was collected by filtration, washed with Diethyl ether (2.0 L) and dried under vacuum at 45°C for 24 h to afford Batch 0057-001 (299.2g, 84% yield without considering the 46g extra batches addition) as a white solid.

**1H NMR (DMSO-d6)** - A residual level of 1.9% of iPrOAc was detected by NMR and extending drying periods under very low vacuum or at very high temperature (up to 100°C) did not provide any reduction in the residual solvent level.

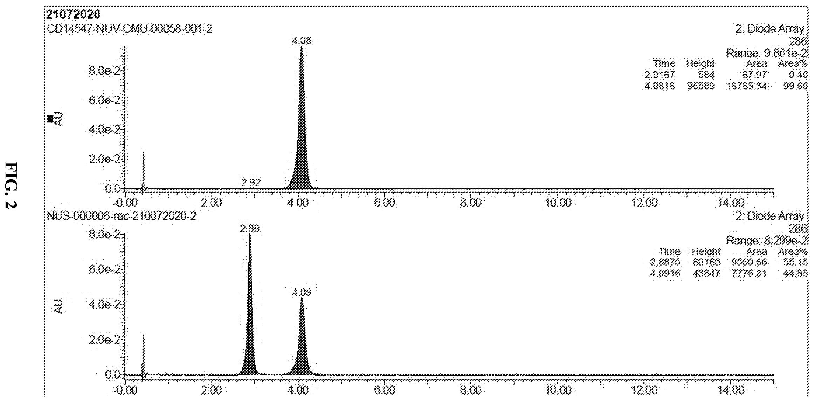

This Batch (0057-001, 257.2g) was dissolved in acetone (16.0 L) under reflux. Then acetone was partially evaporated to a residual volume of c.a. 3.0 L (partial crystallization occurred). 3.0 L of EtOAc were added and 3.0 L of solvent (acetone) were evaporated. 3.0 L of EtOAc were added and evaporation under reduced pressure was pursued. 2.0 L of solvent were evaporated and then 1.0 L of EtOAc was added. The suspension was stirred at 45°C for 18 h and cooled to 23°C, collected by filtration and dried under vacuum at 45°C for 24 h to afford (*S*)-3-(2-aminobenzo[d]oxazol-5-yl)-1-(4-methylpentan-2-yl)-1H-pyrazolo[3,4-d]pyrimidine-4,6-diamine (Compound A) (261.9g, 88% yield) as a white solid. **Fig. 2** shows the SFC separation and analysis of (S)-3-(2-aminobenzo[d]oxazol-5-yl)-1-(4-methylpentan-2-yl)-1H-pyrazolo[3,4-d]pyrimidine-4,6-diamine (Compound A).

The embodiments, illustratively described herein may suitably be practiced in the absence of any element or elements, limitation or limitations, not specifically disclosed herein. Thus, for example, the terms "comprising," "including," "containing," etc. shall be read expansively and without limitation. Additionally, the terms and expressions employed herein have been used as terms of description and not of limitation, and there is no intention in the use of such terms and expressions of excluding any equivalents of the features shown and described or portions thereof, but it is recognized that various modifications are possible within the scope of the claimed technology. Additionally, the phrase "consisting essentially of" will be understood to include those elements specifically recited and those additional elements that do not materially affect the basic and novel characteristics of the claimed technology. The phrase "consisting of" excludes any element not specified.

The present disclosure is not to be limited in terms of the particular embodiments described in this application. Many modifications and variations can be made without departing from its spirit and scope, as will be apparent to those skilled in the art. Functionally equivalent methods and compositions within the scope of the disclosure, in addition to those enumerated herein, will be apparent to those skilled in the art from the foregoing descriptions. Such modifications and variations are intended to fall within the scope of the appended claims. The present disclosure is to be limited only by the terms of the appended claims, along with the full scope of equivalents to which such claims are entitled. It is to be understood that this disclosure is not limited to particular methods, reagents, compounds compositions or biological systems, which can of course vary. It is also to be understood that the terminology used herein is for the purpose of describing particular embodiments only, and is not intended to be limiting.

In addition, where features or aspects of the disclosure are described in terms of Markush groups, those skilled in the art will recognize that the disclosure is also thereby described in terms of any individual member or subgroup of members of the Markush group.

As will be understood by one skilled in the art, for any and all purposes, particularly in terms of providing a written description, all ranges disclosed herein also encompass any and all possible subranges and combinations of subranges thereof. Any listed range can be easily recognized as sufficiently describing and enabling the same range being broken down into at least equal halves, thirds, quarters, fifths, tenths, *etc.* As a non-limiting example, each range discussed herein can be readily broken down into a lower third, middle third and upper third, *etc.* As will also be understood by one skilled in the art all language such as "up to," "at least," "greater than," "less than," and the like, include the number recited and refer to ranges which can be subsequently broken down into subranges as discussed above. Finally, as will be understood by one skilled in the art, a range includes each individual member.

While certain embodiments have been illustrated and described, it should be understood that changes and modifications can be made therein in accordance with ordinary skill in the art without departing from the technology in its broader aspects as defined in the following claims.

## Claims

1. A process of preparing a compound represented by the structural Formula I: 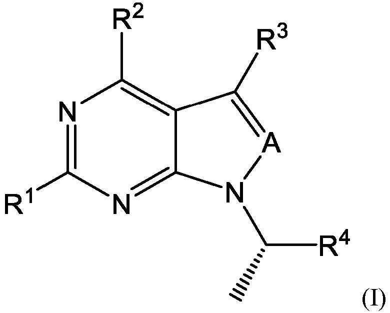 or a hydrate, solvate, or pharmaceutically acceptable salt thereof, comprising:
reacting a compound of Formula II or a salt thereof: 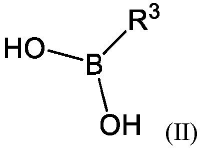
with a compound of Formula III or a salt thereof: 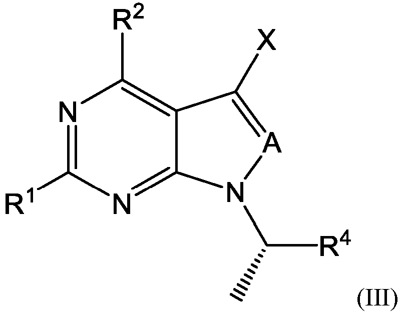
under Suzuki coupling conditions in the presence of a first base to generate a compound of Formula I;
wherein:
X represents F, Cl, Br or I;
A represents a -CH radical or a nitrogen atom;
R¹ represents an -NH₂, -NHMe or -NHEt radical;
R² represents a hydrogen atom, a halogen atom chosen from F, Cl, Br and I,
an -NH₂, -NHalkyl, -NHAc, nitrile, methyl (Me), ethyl (Et), trifluoromethyl, -OH or methoxy radical;
wherein when R ₂ is other than an -NH ₂ radical, then R ₁ represents an -NH ₂ radical;
R³ represents a simple or fused bicyclic aromatic or heteroaromatic radical, which is unsubstituted or mono- or polysubstituted with one or more radicals R^{a}; wherein R^{a} is selected from a halogen atom chosen from F, Cl, Br and I, -NH₂, -NHR⁵, nitrile, methyl, ethyl, trifluoromethyl, -OR⁶;
R⁵ represents hydrogen atom, a radical selected from cyclopropyl, acyl, saturated or unsaturated C₁-C₆ alkyl, optionally interrupted with a heteroatom O or S, and unsubstituted or substituted with a C₃-C₅ cycloalkyl or heterocycloalkyl; and
R⁶ represents a hydrogen atom or a methyl radical;
R⁴ represents a hydrogen atom, a linear or branched C₁-C₁₀ alkyl radical, a saturated or unsaturated C₃-C₁₀ ring or bicycle, optionally interrupted with one or more heteroatoms O, S and N, and unsubstituted or substituted with a radical selected from sulfone, fluoro, cyano, ester, -NR⁷, -NR⁷R⁸, C₃-C₆ cycloalkyl or heterocycloalkyl, or an aromatic ring or a heterocycle which is unsubstituted or mono- or polysubstituted with a halogen atom chosen from Cl and F or a radical selected from -OH, -OMe, trifluoromethyl, methyl, ethyl, -NH₂, -NHMe, -NMe₂; and
R⁷ and R⁸ representing, independently of each other, a hydrogen atom, a C₁-C₃ alkyl, cyclopropyl or acyl radical, or together forming a C₃-C₅ ring; and
optionally converting compound of Formula I to a hydrate, solvate, or pharmaceutically acceptable salt thereof;
wherein the process further comprises preparing the compound of Formula III or a salt thereof by alkylating a compound of Formula (IV): 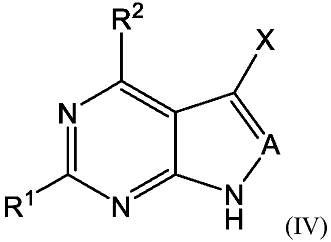 with compound of Formula V: 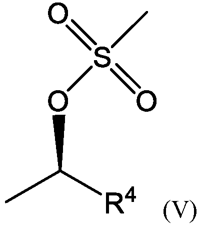 in an anhydrous solvent containing a second base.

2. The process according to claim 1, wherein the compound of Formula II is a hydrochloride salt, and/or wherein the compound of Formula III is an oxalate salt.

3. The process according to claim 1 or claim 2, wherein X is I; and/or wherein A is a nitrogen atom; and/or wherein each of R¹ and R² is -NH₂; and/or wherein R⁴ is an isopropyl radical.

4. The process according to any one of claims 1-3, wherein R³ is

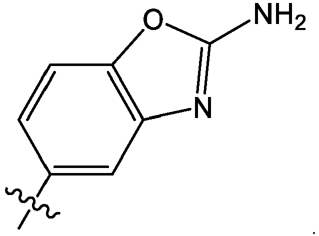

5. The process according to any one of claims 1-4, wherein the Suzuki coupling conditions comprise heating a reaction mixture comprising the compound of Formula II or a salt thereof, the compound of Formula III or a salt thereof, a Suzuki coupling catalyst, the first base and a solvent;
optionally wherein the Suzuki coupling catalyst is selected from the group consisting of PdCl₂(PPh₃)₂, Pd(PPh₃)₄, PdCl₂(dppf)₂, Pd(OAc)₂, Pd₂(dba)₃, or a combination of any two or more thereof, preferably wherein the Suzuki coupling catalyst is Pd(PPh₃)₄;
and/or
wherein the first base is selected from the group consisting of potassium carbonate, sodium carbonate, potassium bicarbonate, tripotassium phosphate, trisodium phosphate, dipotassium hydrogen phosphate, disodium hydrogen phosphate, or a combination of any two or more thereof; preferably wherein the first base is tripotassium phosphate;
and/or
wherein the solvent comprises water and isopropyl alcohol.

6. The process according to any one of claims 1-5, wherein the Suzuki coupling reaction is conducted at a temperature of from about 60 °C to about 120; preferably from about 70 °C to about 100 °C.

7. The process according to any one of claims 1-6, wherein the compound of Formula II is a compound of Formula IIb: 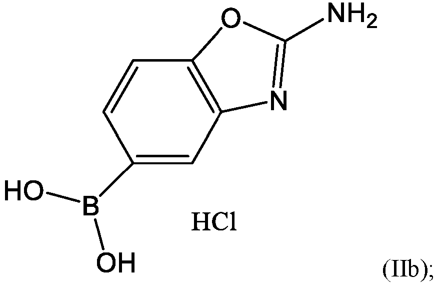 and/or wherein the compound of Formula III is a compound of Formula IIIc: 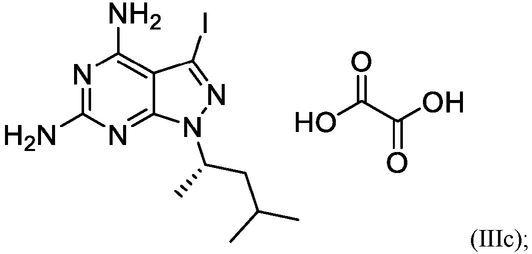 and/or wherein the compound of Formula I is a compound of Formula Ic: 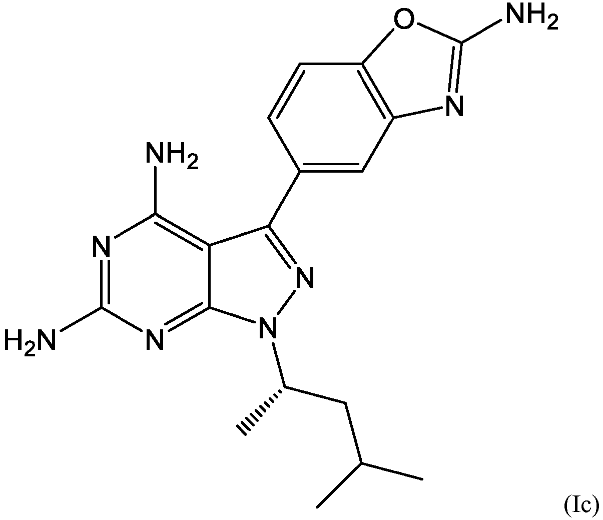 or a hydrate, solvate, or pharmaceutically acceptable salt thereof.

8. The process according to claim 1, wherein the second base is selected from the group consisting of cesium carbonate, cesium bicarbonate, cesium hydroxide potassium carbonate, cesium hydride, or a combination of any two or more thereof, preferably wherein the second base is cesium carbonate.

9. The process according to any one of claims 1-8, wherein the anhydrous solvent is selected from the group consisting of dimethyl sulfoxide, dimethylformamide, dimethylacetamide, N-Methyl-2-pyrrolidone and tetramethylene sulfone or a combination of any two or more thereof.

10. The process according to any one of claims 1-9, wherein the alkylation reaction is conducted at a temperature of from about 20 °C to about 70 °C, preferably from about 30 °C to about 50 °C; and optionally wherein the alkylation reaction is performed for a period of about 5 h to about 36 h, preferably about 10 h to about 24 h.

11. The process according to claim 1, further comprising reacting the compound of Formula III with an acid in a suitable solvent to generate an acid addition salt of compound of Formula III and optionally isolating the acid addition salt of compound of Formula III;
optionally wherein the acid is oxalic acid and the acid addition salt is the oxalate salt of compound of Formula III;
optionally wherein the solvent is selected from the group consisting of ethanol, methanol, isopropanol, butanol, cyclopentyl methylester, methyl acetate, ethyl acetate, isopropyl acetate and mixtures of two or more thereof; and
optionally wherein the reaction is conducted at a temperature ranging from 20°C to 70°C.

12. The process according to claim 1, wherein the compound of Formula V is prepared by reacting an alcohol compound of Formula VI: 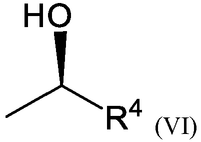 with substituted sulfonyl halide of Formula IX: 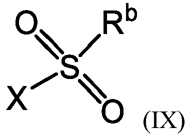 in an organic solvent in the presence of a tertiary amine to generate a compound of Formula V;
wherein:
R⁴ represents a hydrogen atom, a linear or branched C₁-C₁₀ alkyl radical, a saturated or unsaturated C₃-C₁₀ ring or bicycle, optionally interrupted with one or more heteroatoms O, S and N, and unsubstituted or substituted with a radical selected from sulfone, fluoro, cyano, ester, -NR⁷, -NR⁷R⁸, C₃-C₆ cycloalkyl or heterocycloalkyl, or an aromatic ring or a heterocycle which is unsubstituted or mono- or polysubstituted with a halogen atom chosen from Cl and F or a radical selected from -OH, -OMe, trifluoromethyl, methyl, ethyl, -NH₂, -NHMe, -NMe₂; and
R⁷ and R⁸ representing, independently of each other, a hydrogen atom, a C₁-C₃ alkyl, cyclopropyl or acyl radical, or together forming a C₃-C₅ ring; and
R^{b} represents a hydrogen atom, a linear or branched C₁-C₁₀ alkyl radical or a C₁-C₆ aryl radical.

13. The process according to claim 12, wherein R⁴ is an isopropyl radical; and/or wherein R^{b} is methyl.

14. The process according to claim 12 or claim 13, wherein the organic solvent is selected from the group consisting of dimethylacetamide, dimethylformamide, toluene, dimethyl sulfoxide, acetonitrile, tetrahydrofuran, dichloromethane, ethyl acetate, methyl ethyl ketone, acetone, and dioxane, or a mixture of any two or more thereof.

15. The process according to any one of claims 12-14, wherein the tertiary amine is selected from the group consisting of trimethylamine, triethylamine, diisopropylethylamine, dimethylaniline, diethylaniline and dimethylbenzylamine, or a combination of any two or more thereof.

## Patentansprüche

1. Verfahren zur Herstellung einer Verbindung, die durch die Strukturformel I dargestellt wird: 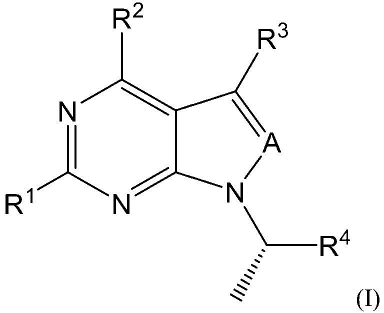 oder eines Hydrats, Solvats oder pharmazeutisch akzeptablen Salzes davon, umfassend:
Reagieren einer Verbindung der Formel II oder eines Salzes davon: 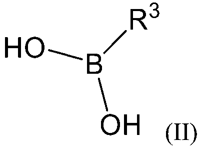 mit einer Verbindung der Formel III oder einem Salz davon: 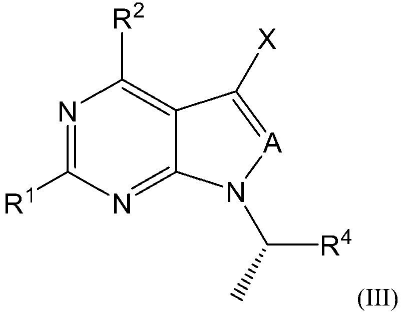 unter Suzuki-Kupplungsbedingungen in Gegenwart einer ersten Base, um eine Verbindung der Formel I zu generieren,
wobei:
X für F, Cl, Br oder I steht;
A für einen -CH-Radikal oder ein Stickstoffatom steht;
R¹ für ein -NH₂-, -NHMₑ- oder -NHEt-Radikal steht;
R² für ein Wasserstoffatom, ein Halogenatom, ausgewählt aus F, Cl, Br und I,
ein -NH₂-, -NHalkyl-, -NHAc-, Nitril-, Methyl- (Me), Ethyl- (Et), Trifluormethyl-, -OH- oder Methoxyradikal;
wobei, wenn R₂ kein -NH ₂-Radikal, R ₁ ist, dann repräsentiert R₁ ein -NH ₂-Radikal;
R³ einen einfachen oder kondensierten biecyclischen aromatischen oder heteroaromatischen Radikal darstellt, der unsubstantiiert oder mit einem oder mehreren Radikalen R\ mono- oder polysubstituiert ist, wobei R^{a} ausgewählt ist aus einem Halogenatom, ausgewählt aus F, Cl, Brand 1, -NH₂, -NHR⁵, Nitril, Methyl, Ethyl, Trifluormethyl, -OR⁶;
R⁵ repräsentiert ein Wasserstoffatom, ein Radikal aus der Gruppe Cyclopropyl, Acyl, gesättigtes oder ungesättigtes C₁-C₆-Alkyl, gegebenenfalls unterbrochen durch ein Heteroatom O oder S, und unsubstituiert oder substituiert durch ein C₃-C₅ Cycloalkyl oder Heterocycloalkyl; und
R⁶ ist ein Wasserstoffatom oder einen Methylrest;
R⁴ steht für ein Wasserstoffatom, einen linearen oder verzweigten C₁-C₁₀-Alkylradikal, einen gesättigten oder ungesättigten C₃-C₁₀-Ring oder ein Bicyclo, gegebenenfalls unterbrochen durch ein oder mehrere
Heteroatome 0, S und N, und unsubstituiert oder substituiert mit einem Radikal, ausgewählt aus Sulfon, Fluor, Cyano, Ester, -NR⁷ -NR⁷ R⁸ , C₃-C₆-Cycloalkyl oder Heterocycloalkyl, oder ein aromatischer Ring oder einen Heterocyclus, der unsubstituiert oder ein- oder mehrfach mit einem Halogenatom, ausgewählt aus Cl und F, oder einem Radikal, ausgewählt aus -OH, -OMe, Trifluormethyl, Methyl, Ethyl, -NH₂, -NHMe, -NMe₂, substituiert ist; und
R⁷ und R⁸ unabhängig voneinander ein Wasserstoffatom, einen C₁-C₃-Alkyl-, Cyclopropyl- oder Acylradikal darstellen oder zusammen einen *C₃-C₅*-Ring bilden; und optional die Verbindung der Formel I in ein Hydrat, Solvat oder pharmazeutisch akzeptables Salz davon umwandeln;
wobei das Verfahren ferner die Herstellung der Verbindung der Formel III oder eines Salzes dieser durch Alkylierung einer Verbindung der Formel (IV) umfasst: 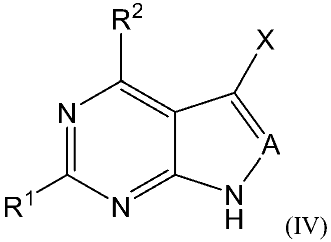 Mit einer Verbindung der Formel V: 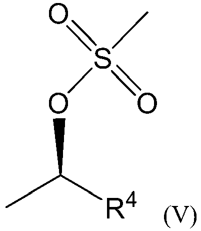 in einem wasserfreien Lösungsmittel, das eine zweite Base enthält.

2. Verfahren nach Anspruch 1, wobei die Verbindung der Formel II ein Hydrochloridsalz ist und/oder wobei die Verbindung der Formel III ein Oxalatsalz ist.

3. Verfahren nach Anspruch 1 oder Anspruch 2, wobei X I ist; und/oder wobei A ein Stickstoffatom ist; und/oder wobei jedes R¹ und R² -NH₂ ist; und/oder wobei R⁴ ein Isopropylrest ist.

4. Verfahren nach einem der Ansprüche 1 bis 3, wobei R³ ist.

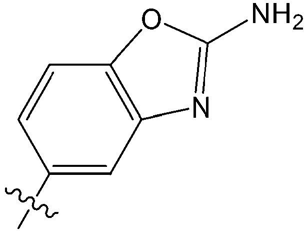

5. Verfahren nach einem der Ansprüche 1 bis 4, wobei die Suzuki-Kupplungsbedingungen das Erhitzen eines Reaktionsgemisches umfassen, das die Verbindung der Formel II oder ein Salz davon, die Verbindung der Formel III oder ein Salz davon, einen Suzuki-Kupplungskatalysator, die erste Base und ein Lösungsmittel umfasst;
optional, wobei der Suzuki-Kupplungskatalysator aus der Gruppe ausgewählt ist, die aus PdCl₂(PPh₃)₂, Pd(PPh₃)₄, PdCl₂(dppf)₂, Pd(OAc)₂, Pd₂(dba)₃ oder einer Kombination aus zwei oder mehr davon besteht, wobei der Suzuki-Kupplungskatalysator vorzugsweise Pd(PPh₃)₄ ist; und/oder wobei die erste Base aus der Gruppe ausgewählt ist, die aus Kaliumcarbonat, Natriumcarbonat, Kaliumbicarbonat, Trikaliumphosphat, Trinatriumphosphat, Dikaliumhydrogenphosphat, Dinatriumhydrogenphosphat oder einer Kombination aus zwei oder mehr davon besteht; vorzugsweise wobei die erste Base Trikaliumphosphat ist;und/oder wobei das Lösungsmittel Wasser und Isopropylalkohol umfasst.

6. Verfahren nach einem der Ansprüche 1 bis 5, wobei die Suzuki-Kupplungsreaktion bei einer Temperatur von etwa 60 °C bis etwa 120 °C, vorzugsweise von etwa 70 °C bis etwa 100 °C, durchgeführt wird.

7. Verfahren nach einem der Ansprüche 1 bis 6, wobei die Verbindung der Formel II eine Verbindung der Formel IIb ist: 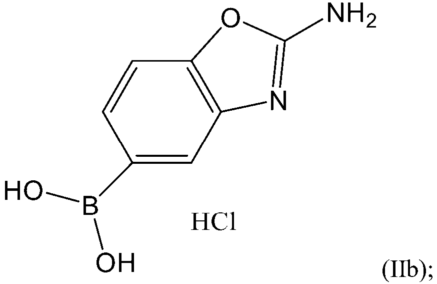 und/oder wobei die Verbindung der Formel III eine Verbindung der Formel IIIc ist: 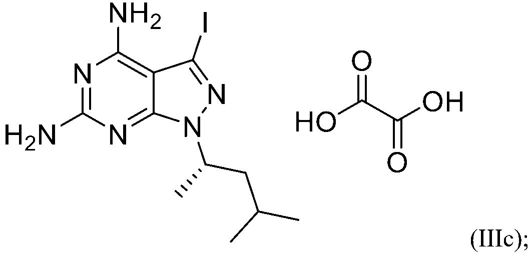 und/oder wobei die Verbindung der Formel I eine Verbindung der Formel Ic ist: 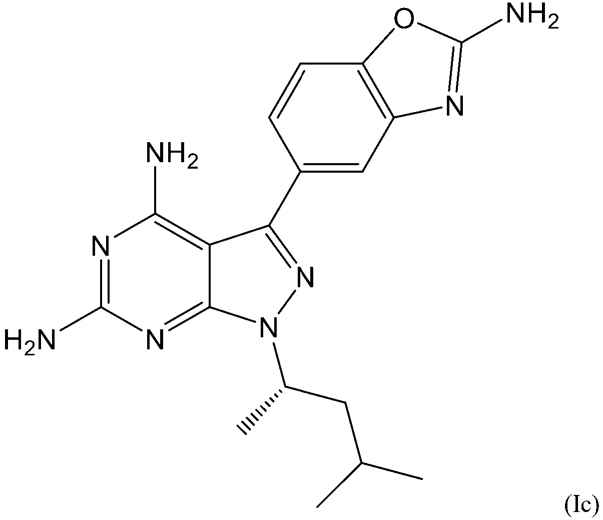 oder ein Hydrat, Solvat oder pharmazeutisch verträgliches Salz davon.

8. Verfahren nach Anspruch 1, wobei die zweite Base aus der Gruppe ausgewählt ist, die aus Cäsiumcarbonat, Cäsiumbicarbonat, Cäsiumhydroxid, Kaliumcarbonat, Cäsiumhydrid oder einer Kombination aus zwei oder mehr davon besteht, wobei die zweite Base vorzugsweise Cäsiumcarbonat ist.

9. Verfahren nach einem der Ansprüche 1 bis 8, wobei das wasserfreie Lösungsmittel ausgewählt ist aus der Gruppe bestehend aus Dimethylsulfoxid, Dimethylformamid, Dimethylacetamid, N-Methyl-2-pyrrolidon und Tetramethylensulfon oder eine Kombination aus zwei oder mehr davon.

10. Verfahren nach einem der Ansprüche 1 bis 9, wobei die Alkylierungsreaktion bei einer Temperatur von etwa 20 °C bis etwa 70 °C, vorzugsweise von etwa 30 °C bis etwa 50 °C, durchgeführt wird; und wobei die Alkylierungsreaktion optional über einen Zeitraum von etwa 5 Stunden bis etwa 36 Stunden, vorzugsweise etwa 10 Stunden bis etwa 24 Stunden, durchgeführt wird.

11. Verfahren nach Anspruch 1, das ferner das Umsetzen der Verbindung der Formel III mit einer Säure in einem geeigneten Lösungsmittel umfasst, um ein Säureadditionssalz der Verbindung der Formel III zu erzeugen, und gegebenenfalls das Isolieren des Säureadditionssalzes der Verbindung der Formel III; wobei gegebenenfalls die Säure Oxalsäure ist und das Säureadditionssalz das Oxalatsalz der Verbindung der Formel III ist; wobei das Lösungsmittel gegebenenfalls aus der Gruppe ausgewählt ist, die aus Ethanol, Methanol, Isopropanol, Butanol, Cyclopentylmethylester, Methylacetat, Ethylacetat, Isopropylacetat und Mischungen aus zwei oder mehr davon besteht; und wobei die Reaktion gegebenenfalls bei einer Temperatur im Bereich von 20 °C bis 70 °C durchgeführt wird.

12. Verfahren nach Anspruch 1, wobei die Verbindung der Formel V durch Umsetzung einer Alkoholverbindung der Formel VI hergestellt wird: 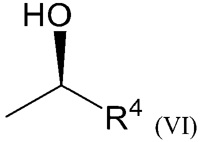 mit substituiertem Sulfonylhalogenid der Formel IX: 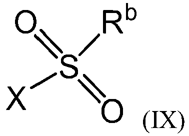 in einem organischen Lösungsmittel in Gegenwart eines tertiären Amins, um eine Verbindung der Formel V zu erzeugen; wobei:
R⁴ ein Wasserstoffatom, einen linearen oder verzweigten C₁-C₁₀-Alkylrest, einen gesättigten oder ungesättigten C₃-C₁₀-Ring oder Bicyclus, gegebenenfalls unterbrochen durch ein oder mehrere Heteroatome 0, S und N, und unsubstituiert oder substituiert mit einem Rest, ausgewählt aus
Sulfon, Fluor, Cyano, Ester, -NR⁷ -NR⁷R⁸ C₃-C₆-Cycloalkyl oder Heterocycloalkyl, oder einem
aromatischen Ring oder einen Heterocyclus, der unsubstituiert oder ein- oder mehrfach mit einem Halogenatom, ausgewählt aus Cl und F, oder einem Rest, ausgewählt aus - OH, -OMe, Trifluormethyl, Methyl, Ethyl, -NH₂, -NHMe, -NMe₂, substituiert ist; und R⁷ und R⁸ unabhängig voneinander ein Wasserstoffatom, einen C₁-C₃-Alkyl-, Cyclopropyl- oder Acylrest darstellen oder zusammen einen *C₃-C₅*-Ring bilden; und R^{b} ein Wasserstoffatom, einen linearen oder verzweigten C₁-C₁₀-Alkylrest oder einen C₁-C₆-Arylrest darstellt.

13. Verfahren nach Anspruch 12, wobei R⁴ ein Isopropylrest ist; und/oder wobei R^{b} Methyl ist.

14. Verfahren nach Anspruch 12 oder Anspruch 13, wobei das organische Lösungsmittel aus der Gruppe ausgewählt ist, die aus Dimethylacetamid, Dimethylformamid, Toluol, Dimethylsulfoxid, Acetonitril, Tetrahydrofuran, Dichlormethan, Ethylacetat, Methylethylketon, Aceton und Dioxan oder einer Mischung aus zwei oder mehr davon besteht.

15. Verfahren nach einem der Ansprüche 12 bis 14, wobei das tertiäre Amin aus der Gruppe ausgewählt ist, die aus Trimethylamin, Triethylamin, Disopropylethylamin, Dimethylanilin, Diethylanilin und Dimethylbenzylamin oder einer Kombination aus zwei oder mehr davon besteht.

## Revendications

1. Procédé de préparation d'un composé représenté par la formule structurelle I : 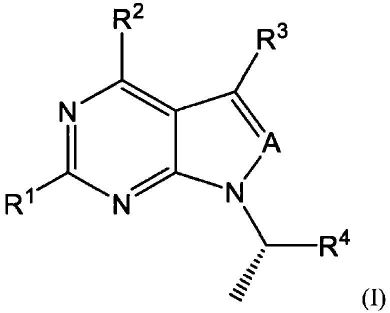 ou d'un hydrate, d'un solvate ou d'un sel pharmaceutiquement acceptable de celui-ci, comprenant :
la réaction d'un composé de formule II ou d'un sel de celui-ci : 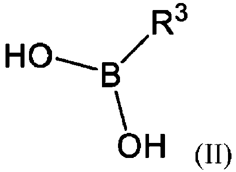
avec un composé de formule III ou un sel de celui-ci : 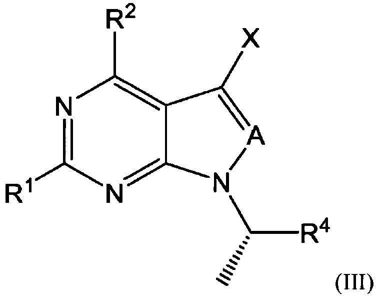
dans des conditions de couplage de Suzuki en présence d'une première base pour générer un composé de formule I ;
dans lequel :
X représente F, Cl, Br ou I ;
A représente un radical -CH ou un atome d'azote ;
R¹ représente un radical -NH₂, -NHMe ou -NHEt ;
R² représente un atome d'hydrogène, un atome d'halogène choisi parmi F, Cl, Br et I, un radical -NH₂, -NHalkyle, - NHAc, nitrile, méthyle (Me), éthyle (Et), trifluorométhyle, -OH ou méthoxy ;
dans lequel lorsque R² est autre qu'un radical -NH₂, alors R¹ représente un radical -NH₂ ;
R³ représente un radical aromatique ou hétéroaromatique bicyclique simple ou condensé, qui est non substitué ou mono- ou poly-substitué avec un ou plusieurs radicaux R^{a} ; dans lequel R^{a} est sélectionné parmi un atome d'halogène choisi parmi F, Cl, Br et I, -NH₂, -NHR⁵, un nitrile, un méthyle, un éthyle, un trifluorométhyle, -OR⁶ ;
R⁵ représente un atome d'hydrogène, un radical sélectionné parmi un cyclopropyle, un acyle, un alkyle saturé ou insaturé en C₁-C₆, facultativement interrompu par un hétéroatome O ou S, et
non substitué ou substitué avec un cycloalkyle ou hétérocycloalkyle en C₃-C₅ ; et
R⁶ représente un atome d'hydrogène ou un radical méthyle ; R⁴ représente un atome d'hydrogène, un radical alkyle linéaire ou ramifié en C₁-C₁₀, un cycle ou bicycle en C₃-C₁₀ saturé ou insaturé, facultativement interrompu par un ou plusieurs hétéroatomes O, S et N, et non substitué ou substitué avec un radical sélectionné parmi une sulfone, un fluoro, un cyano, un ester, -NR⁷, -NR⁷R⁸, un cycloalkyle ou hétérocycloalkyle en C₃-C₆ , ou un cycle aromatique ou un hétérocycle qui est non substitué ou mono- ou poly-substitué avec un atome d'halogène choisi parmi Cl et F ou un radical sélectionné parmi -OH, -OMe, un trifluorométhyle, un méthyle, un éthyle, -NH₂, -NHMe, - NMe₂ ; et
R⁷ et R⁸ représentent, indépendamment l'un de l'autre, un atome d'hydrogène, un radical alkyle en C₁-C₃, cyclopropyle ou acyle, ou forment ensemble un cycle en C₃-C₅ ; et
facultativement la conversion d'un composé de formule I en un hydrate, un solvate ou un sel pharmaceutiquement acceptable de celui-ci ;
dans lequel le procédé comprend en outre la préparation du composé de formule III ou d'un sel de celui-ci par alkylation d'un composé de formule (IV) : 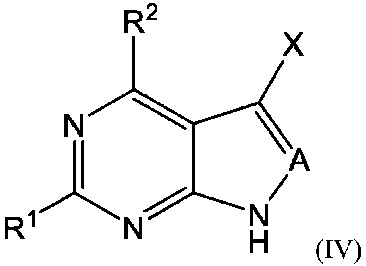 avec un composé de formule V : 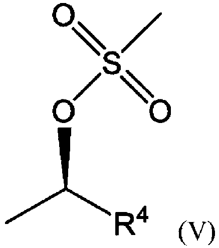 dans un solvant anhydre contenant une seconde base.

2. Procédé selon la revendication 1, dans lequel le composé de formule II est un sel de chlorhydrate et/ou dans lequel le composé de formule III est un sel d'oxalate.

3. Procédé selon la revendication 1 ou la revendication 2, dans lequel X est I ; et/ou dans lequel A est un atome d'azote ; et/ou dans lequel chacun parmi R¹ et R² est - NH₂ ; et/ou dans lequel R⁴ est un radical isopropyle.

4. Procédé selon l'une quelconque des revendications 1 à 3, dans lequel R³ est

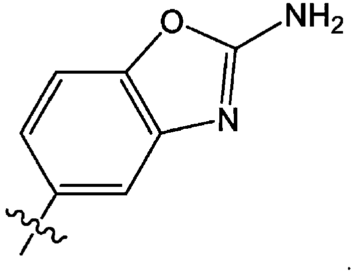

5. Procédé selon l'une quelconque des revendications 1 à 4, dans lequel les conditions de couplage de Suzuki comprennent le chauffage d'un mélange réactionnel comprenant le composé de formule II ou un sel de celui-ci, le composé de formule III ou un sel de celui-ci, un catalyseur de couplage de Suzuki, la première base et un solvant ;
facultativement dans lequel le catalyseur de couplage de Suzuki est sélectionné dans le groupe consistant en PdCl₂(PPh₃)₂, Pd(PPh₃)₄, PdCl₂(dppf)₂, Pd(OAc)₂, Pd₂(dba)₃, ou une combinaison de deux ou plus de ceux-ci, de préférence dans lequel le catalyseur de couplage de Suzuki est Pd(PPh₃)₄ ;
et/ou
dans lequel la première base est sélectionnée dans le groupe consistant en le carbonate de potassium, le carbonate de sodium, le bicarbonate de potassium, le phosphate tripotassique, le phosphate trisodique, l'hydrogénophosphate dipotassique, l'hydrogénophosphate disodique, ou une combinaison de deux ou plus de ceux-ci ; de préférence, dans lequel la première base est le phosphate tripotassique ;
et/ou
dans lequel le solvant comprend de l'eau et de l'alcool isopropylique.

6. Procédé selon l'une quelconque des revendications 1 à 5, dans lequel la réaction de couplage de Suzuki est effectuée à une température d'environ 60 °C à environ 120 °C ; de préférence d'environ 70 °C à environ 100 °C.

7. Procédé selon l'une quelconque des revendications 1 à 6, dans lequel le composé de formule II est un composé de formule IIb : 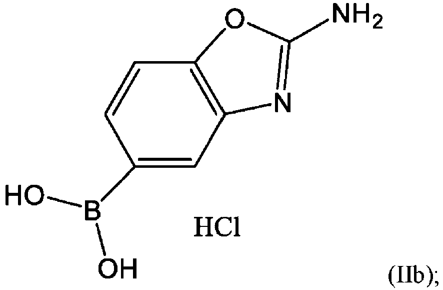 et/ou dans lequel le composé de formule III est un composé de formule IIIc : 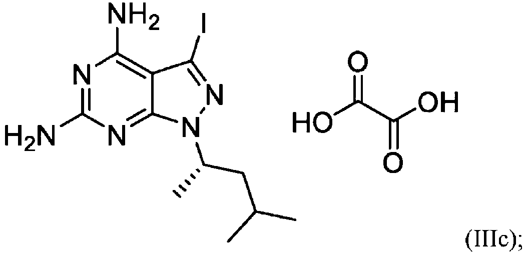 et/ou dans lequel le composé de formule I est un composé de formule Ic : 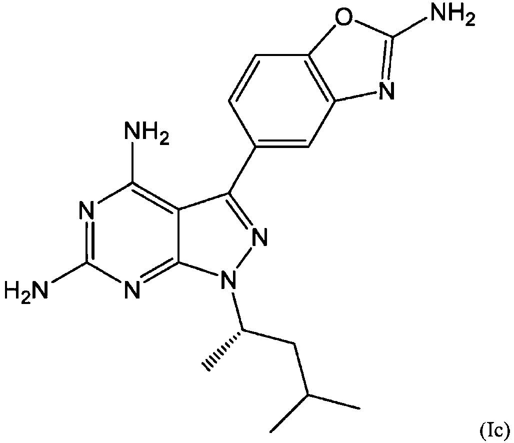 ou un hydrate, un solvate ou un sel pharmaceutiquement acceptable de ceux-ci.

8. Procédé selon la revendication 1, dans lequel la seconde base est sélectionnée dans le groupe consistant en le carbonate de césium, le bicarbonate de césium, l'hydroxyde de césium, le carbonate de potassium, l'hydrure de césium, ou une combinaison de deux ou plus de ceux-ci, de préférence dans lequel la seconde base est le carbonate de césium.

9. Procédé selon l'une quelconque des revendications 1 à 8, dans lequel le solvant anhydre est sélectionné dans le groupe consistant en le diméthylsulfoxyde, le diméthylformamide, le diméthylacétamide, la N-méthyl-2-pyrrolidone et la tétraméthylène-sulfone ou une combinaison de deux ou plus de ceux-ci.

10. Procédé selon l'une quelconque des revendications 1 à 9, dans lequel la réaction d'alkylation est effectuée à une température d'environ 20 °C à environ 70 °C, de préférence d'environ 30 °C à environ 50 °C ; et facultativement dans lequel la réaction d'alkylation est effectuée pendant une période d'environ 5 h à environ 36 h, de préférence d'environ 10 h à environ 24 h.

11. Procédé selon la revendication 1, comprenant en outre la réaction du composé de formule III avec un acide dans un solvant approprié afin de générer un sel d'addition d'acide du composé de formule III et facultativement l'isolement du sel d'addition d'acide du composé de formule III ;
facultativement dans lequel l'acide est l'acide oxalique et le sel d'addition d'acide est le sel d'oxalate du composé de la formule III ;
facultativement dans lequel le solvant est sélectionné dans le groupe consistant en l'éthanol, le méthanol, l'isopropanol, le butanol, le cyclopentylméthylester, l'acétate de méthyle, l'acétate d'éthyle, l'acétate d'isopropyle et des mélanges de deux ou plus de ceux-ci ; et
facultativement dans lequel la réaction est effectuée à une température allant de 20 °C à 70 °C.

12. Procédé selon la revendication 1, dans lequel le composé de formule V est préparé en faisant réagir un composé de type alcool de formule VI : 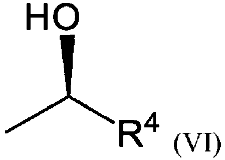 avec un halogénure de sulfonyle substitué de formule IX : 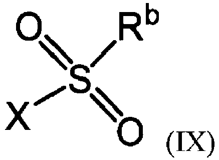 dans un solvant organique en présence d'une amine tertiaire afin de générer un composé de formule V ;
dans lequel
R⁴ représente un atome d'hydrogène, un radical alkyle linéaire ou ramifié en C₁-C₁₀, un cycle ou bicycle en C₃-C₁₀ saturé ou insaturé, facultativement interrompu par un ou plusieurs hétéroatomes O, S et N, et non substitué ou substitué avec un radical sélectionné parmi une sulfone, un fluoro, un cyano, un ester, -NR⁷, -NR⁷R⁸, un cycloalkyle ou hétérocycloalkyle en C₃-C₆ , ou un cycle aromatique ou un hétérocycle qui est non substitué ou mono- ou poly-substitué avec un atome d'halogène choisi parmi Cl et F ou un radical sélectionné parmi -OH, -OMe, un trifluorométhyle, un méthyle, un éthyle, -NH₂, -NHMe, - NMe₂ ; et
R⁷ et R⁸ représentent, indépendamment l'un de l'autre, un atome d'hydrogène, un radical alkyle en C₁-C₃, cyclopropyle ou acyle, ou forment ensemble un cycle en C₃-C₅ ; et
R^{b} représente un atome d'hydrogène, un radical alkyle linéaire ou ramifié en C₁-C₁₀, ou un radical aryle en C₁-C₆.

13. Procédé selon la revendication 12, dans lequel R⁴ est un radical isopropyle ; et/ou dans lequel R^{b} est un méthyle.

14. Procédé selon la revendication 12 ou la revendication 13, dans lequel le solvant organique est sélectionné dans le groupe consistant en le diméthylacétamide, le diméthylformamide, le toluène, le diméthylsulfoxyde, l'acétonitrile, le tétrahydrofurane, le dichlorométhane, l'acétate d'éthyle, la méthyléthylcétone, l'acétone et le dioxane, ou un mélange de deux ou plus de ceux-ci.

15. Procédé selon l'une quelconque des revendications 12 à 14, dans lequel l'amine tertiaire est sélectionnée dans le groupe consistant en la triméthylamine, la triéthylamine, la diisopropyléthylamine, la diméthylaniline, la diéthylaniline et la diméthylbenzylamine, ou une combinaison de deux ou plus de celles-ci.
